# DEMANDE DE BREVET EUROPEEN

(11) **EP 4 091 618 A1**
(43) Date de publication de la demande: **23.11.2022**
(21) Numéro de dépôt: 22179679.0
(22) Date de dépôt: 28.02.2018
(51) Int. Cl.: A61K 35/28, A61K 35/51, C12N 5/0775, A61P 31/00

(54) **CELLULES SOUCHES MESENCHYMATEUSES ISSUES DE LA GELÉE DE WHARTON POUR LE TRAITEMENT DU SEPSIS**

(30) Priorité: 28.02.2017 FR 1751649
(62) Demande divisionnaire de: 18712945.7
(71) Demandeur: Université de Lorraine, 54052 Nancy (FR); Centre Hospitalier Régional Universitaire de Nancy, 54035 Nancy Cedex (FR)
(72) Inventeur: BENSOUSSAN, Danièle, 54000 Nancy (FR); GIBOT, Sébastien, 54110 Réméréville (FR); REPPEL, Loïc, 54000 Nancy (FR); LAROYE, Caroline, 54000 Nancy (FR); BOUFENZER, Amir, 54600 Villers-lès-Nancy (FR); AVERCENC, Léonore, 54340 Pompey (FR); HUSELSTEIN, Céline, 54550 Bainville-sur-Madon (FR)
(74) Mandataire: Icosa

(57) **Abrégé**

La présente invention concerne des cellules souches mésenchymateuses humaines issues de la gelée de Wharton, leur méthode de préparation et leurs utilisations thérapeutiques, notamment dans le traitement du sepsis.

## Description

### DOMAINE DE L'INVENTION

La présente invention concerne des cellules souches mésenchymateuses humaines issues de la gelée de Wharton, leur méthode de préparation et leurs utilisations thérapeutiques.

### ÉTAT DE LA TECHNIQUE

Bien que souvent méconnu, le choc septique représente, à égalité avec les infarctus du myocarde, la onzième cause de mortalité à travers le monde. Première cause d'admission et de mortalité dans les unités de réanimation non coronariennes, son incidence, comprise entre 50 à 100 cas pour 100 000 habitants, n'a cessé de croître au cours des dernières années (Dombrovskiy *et al.,* 2007). L'augmentation de l'espérance de vie, le nombre croissant de bactéries multi-résistantes et un diagnostic plus fréquemment posé sont à l'origine de ce phénomène.

La physiopathologie du choc septique est particulièrement complexe (Iskander *et al.,* 2013) et implique de façon simultanée à la fois des cytokines pro- et anti-inflammatoires ainsi qu'une immunoparalysie. Il en ressort une absence de traitement spécifique du choc septique due à l'incapacité des molécules pharmaceutiques conventionnelles à agir à la fois sur le versant inflammatoire et sur le versant anti-inflammatoire, tout en relançant le système immunitaire.

Or, de nombreuses études utilisant des modèles murins d'endotoxémies ou de péritonites ont mis en évidence l'aptitude des cellules souches mésenchymateuses (CSM) à diminuer les taux plasmatiques d'IL6, d'IL1β, d'IL 12, d'IL2 et d'IL 17 (Chao *et al.,* 2014a ; Kim *et al.,* 2014 ; Luo *et al.,* 2014 ; Pedrazza *et al.,* 2014) et abaisser la concentration tissulaire du TNFα, de l'IL6, IL1β, IL 12 au niveau pulmonaire, hépatique et intestinal (Gonzalez-Rey *et al.,* 2009), ou au niveau du liquide broncho-alvéolaire (Mei *et al.,* 2010a).

La capacité des CSM à limiter la migration tissulaire des neutrophiles concourt à modérer les effets délétères de l'inflammation sur les tissus (Rojas *et al.,* 2014). Les études des coupes histologiques de souris soumises à un sepsis polymicrobien par ligature et perforation du cæcum (CLP) ou soumises à un sepsis par endotoxémie montrent une diminution de l'inflammation des tissus pulmonaires et rénaux lorsqu'elles ont été traitées avec des CSM (Krasnodembskaya *et al.,* 2010). De même, ces souris présentent des marqueurs biologiques des fonctions d'organes tels que l'amylasémie, la créatininémie, la bilirubinémie, les aspartate aminotransférases (ASAT) et les alanine aminotransférases (ALAT) significativement meilleurs que les souris traitées par une solution saline (Luo *et al.,* 2014 ; Pedrazza *et al.,* 2014).

Outre leur capacité à limiter l'altération tissulaire, les CSM sont capables d'augmenter la clairance bactérienne au cours d'un choc septique. En effet, plusieurs équipes ont démontré que l'injection de CSM chez des souris septiques aboutissait à la diminution de la bactériémie par augmentation de l'activité phagocytaire des monocytes, macrophages et neutrophiles mais également par synthèse et sécrétion par les CSM elles-mêmes de peptides antibactériens tels que le LL-37 et l'hepcidine (Alcayaga-Miranda *et al.,* 2015 ; Devaney *et al.,* 2015 ; Gonzalez-Rey *et al.,* 2009 ; Hall *et al.,* 2013 ; Krasnodembskaya *et al.,* 2010 ; Mei *et al.,* 2010b).

Par ailleurs les CSM présentent des propriétés intrinsèques de « homing » qui leur confèrent une susceptibilité à migrer, selon un gradient de chimiokines, vers les organes lésés tels que le foie, les reins et les poumons. Cette capacité à rejoindre les tissus endommagés est un atout des CSM particulièrement intéressant dans le cadre d'une pathologie à défaillance d'organes tel que le choc septique (Wannemuehler *et al.,* 2012).

Enfin, la diminution des lésions tissulaires par l'injection de CSM dans des modèles murins de sepsis sévère aboutit à l'augmentation de la survie. De nombreuses études mettent en évidence une diminution significative de la mortalité des animaux traités en pré- ou post-sepsis par des CSM (Alcayaga-Miranda *et al.,* 2015 ; Devaney *et al.,* 2015 ; Gonzalez-Rey *et al.,* 2009 ; Krasnodembskaya *et al.,* 2012 ; Luo *et al.,* 2014 ; Németh *et al.,* 2009). Ainsi, par leurs capacités à réguler l'hyper-inflammation et modérer les lésions tissulaires, les CSM semblent être en mesure d'améliorer nettement la survie post-choc septique.

On distingue trois sources majeures de CSM : la moelle osseuse (MO), le tissu adipeux et la gelée de Wharton (GW) du cordon ombilical. Comparées aux CSM de MO, les CSM de GW obtenues de façon simple, non invasive et sans anesthésie, permettraient de démocratiser le don de CSM en s'affranchissant des risques liés au prélèvement de MO. Cependant, si les CSM de MO ou les CSM du tissu adipeux ont été étudiées dans des modèles animaux du choc septique, le potentiel des CSM issues de la gelée de Wharton dans cette indication reste faiblement documenté.

À ce jour, seules 5 publications ont analysé le potentiel thérapeutique des CSM-GW sur le choc septique en utilisant un modèle murin de CLP (Chao *et al.,* 2014a ; Condor *et al.,* 2016 ; Wu *et al.,* 2015 ; Zhao *et al.,* 2014). Cependant, les cellules utilisées dans ces études sont des cellules fraîches. Aucune étude publiée à ce jour ne concerne des CSM-GW décongelées dans l'indication du choc septique, ceci peut être en raison des connaissances générales sur l'impact de la congélation et de la décongélation sur le potentiel pharmacologique et immunomodulateur des cellules souches (François *et al.,* Cytotherapy. 2012.14(2):147-52 ; Chinnadurai *et al.,* 2016 ; Moll *et al.,* 2016).

Par ailleurs, Mezey et Nemeth (2015) ont décrit une diminution des propriétés antibactériennes des CSM lorsqu'elles sont injectées tardivement.

En conséquence, à ce jour, ces contraintes techniques empêchent l'utilisation des CSM-GW en condition clinique pour un traitement thérapeutique.

Contre toute attente, les Inventeurs de la présente invention montrent pour la première fois que les CSM-GW décongelées conservent leur potentiel thérapeutique et peuvent être utilisées dans le traitement du sepsis.

### RÉSUMÉ

La présente invention concerne des cellules souches mésenchymateuses (CSM) humaines décongelées issues de la gelée de Wharton pour leur utilisation dans le traitement du sepsis, notamment du choc septique.

Dans un mode de réalisation, les CSM humaines décongelées issues de la gelée de Wharton de la présente invention sont caractérisées en ce que le niveau d'expression d'au moins un marqueur sélectionné parmi CD90, CD73, CD105, CD29, CD44, CD146, CD166, HLA-ABC est au moins 10% inférieur au niveau d'expression du même marqueur dans des CSM humaines fraîches issues de la gelée de Wharton.

Dans un mode de réalisation, les CSM humaines décongelées issues de la gelée de Wharton de la présente invention sont caractérisées en ce que le niveau d'expression du marqueur CD90 est au moins 10% inférieur au niveau d'expression du marqueur CD90 dans des CSM humaines fraîches issues de la gelée de Wharton.

Dans un mode de réalisation, les CSM humaines décongelées issues de la gelée de Wharton de la présente invention sont caractérisées en ce que lesdites CSM expriment au moins une protéine sélectionnée parmi le groupe comprenant ACTB, ANXA1, CAPZB, LASP1, PRDX2, PRDX3, PSA3, RS12 et SYWC.

Dans un mode de réalisation, les CSM humaines décongelées issues de la gelée de Wharton de la présente invention sont caractérisées en ce que lesdites CSM n'expriment substantiellement pas au moins une protéine sélectionnée parmi le groupe comprenant ACTS, AL1B1, ANX10, GBB1, GBB2, GPRIN1, DTNA, MIPO1, PSB3 etPSDE.

Dans un mode de réalisation, les CSM humaines décongelées issues de la gelée de Wharton de la présente invention sont caractérisées en ce que lesdites CSM, en condition *in vitro* et/ou en condition non-inflammatoire, sécrètent au moins un facteur de croissance sélectionné parmi BMP-7, IGFBP-1, insuline, FGF-7, NT-4 et VEGF-D.

Dans un mode de réalisation, les CSM humaines décongelées issues de la gelée de Wharton de la présente invention sont caractérisées en ce que lesdites CSM, en condition *in vivo* et/ou en condition inflammatoire, sécrètent au moins un facteur de croissance sélectionné parmi BMP-7 et TGFβ3.

Dans un mode de réalisation, les CSM humaines décongelées issues de la gelée de Wharton de la présente invention sont caractérisées en ce que lesdites CSM, en condition *in vivo* et/ou en condition inflammatoire, ne sécrètent substantiellement pas d'IGFBP-1.

Dans un mode de réalisation, les CSM humaines décongelées issues de la gelée de Wharton de la présente invention sont caractérisées en ce que lesdites CSM sécrètent au moins 1.2 fois plus de VEGF que des CSM humaines fraîches issues de la gelée de Wharton.

Dans un mode de réalisation, les CSM humaines décongelées issues de la gelée de Wharton de la présente invention sont caractérisées en ce que lesdites CSM induisent une augmentation de la concentration sérique de VEGF chez le patient d'au moins 5% par rapport à des CSM humaines fraîches issues de la gelée de Wharton.

Dans un mode de réalisation, les CSM humaines décongelées issues de la gelée de Wharton de la présente invention sont caractérisées en ce que lesdites cellules sont issues d'un tissu de cordon ombilical humain provenant d'une mère répondant à au moins l'un des critères suivants : ayant reçu une administration d'ocytocine lors de l'accouchement, ayant accouché par travail dirigé, ayant accouché à terme, n'ayant pas présenté de prééclampsie pendant la grossesse, dont l'enfant n'a pas présenté de troubles néonataux et ayant subi une imprégnation tabagique au cours de la grossesse.

Dans un mode de réalisation, les CSM humaines décongelées issues de la gelée de Wharton de la présente invention sont caractérisées en ce que lesdites CSM sont des cellules de grade clinique.

Dans un mode de réalisation, les CSM humaines décongelées issues de la gelée de Wharton de la présente invention sont caractérisées en ce que lesdites cellules sont directement issues de la décongélation sans remise en culture après la décongélation.

La présente invention concerne également une composition pharmaceutique comprenant des CSM humaines décongelées issues de la gelée de Wharton selon la présente invention et un excipient pharmaceutiquement acceptable, pour son utilisation dans le traitement du sepsis.

La présente invention concerne également un procédé de préparation de cellules souches mésenchymateuses de grade clinique issues de la gelée de Wharton, comprenant les étapes suivantes :
(i) cultiver un tissu de cordons ombilicaux humains contenant la gelée de Wharton dans un milieu de culture de grade clinique pour l'adhésion de cellules ;
(ii) incuber les cellules adhérentes dans un milieu contenant du lysat plaquettaire ;
caractérisé en ce que ledit tissu provient d'une mère répondant à au moins l'un des critères suivants : ayant reçu une administration d'ocytocine lors de l'accouchement, ayant accouché par travail dirigé, ayant accouché à terme, n'ayant pas présenté de prééclampsie pendant la grossesse, dont l'enfant n'a pas présenté de troubles néonataux et ayant subi une imprégnation tabagique au cours de la grossesse.

### DÉFINITIONS

Dans la présente invention, les termes ci-dessous sont définis de la manière suivante :
On entend par « **absence de troubles néonataux** », le fait qu'un nouveau-né ne présente aucun signe de prématurité, ni de retard de croissance intra-utérin, ni de souffrance fœtale. La prématurité est déterminée par le nombre de semaines d'aménorrhée. Le retard de croissance intra-utérin est déterminé par le poids de naissance, la taille à la naissance et le périmètre crânien. La souffrance fœtale est déterminée par le pH artériel du sang de cordon et le score Apgar défini par A = apparence (coloration), P = pouls, G = grimace (réflexes aux stimuli), A = activité (tonus musculaire), R = respiration (efforts respiratoires). Les seuils de ces paramètres pour déterminer si un nouveau-né ne présente pas de troubles néonataux sont bien connus de l'homme de métier.

On entend par « **accouchement à terme** », un accouchement donnant la naissance d'un nouveau-né ayant un poids de naissance supérieur à 3,2 kg, après au moins 39,5 semaines d'aménorrhée et le poids du placenta au moment de l'accouchement étant supérieur à 550 grammes.

On entend par « **accouchement par travail dirigé** », un accouchement aidé par administration d'ocytocine ou la rupture artificielle de la poche des eaux.

On entend par « **cellules de grade clinique** », des cellules n'ayant eu aucun contact avec une substance d'origine animale non humaine, telle que le sérum animal non humain, pendant l'isolement, la culture, la cryoconservation et la décongélation des cellules. Les cellules de grade clinique sont des cellules produites dans des conditions de culture compatibles avec une utilisation chez l'homme et conforme au Bonnes pratiques de production pharmaceutique.

On entend par « **cellules souches cryoconservées** », des cellules souches conservées à très basse température, de préférence entre -150°C et -196°C, de préférence en présence d'une solution cryoprotectrice renfermant du diméthyl sulfoxyde (DMSO).

On entend par « **cellules souches mésenchymateuses** », des cellules souches multipotentes, d'origine mésodermique, retrouvées dans divers tissus de l'organisme adulte tels que la moelle osseuse, le tissu adipeux, ainsi que le cordon ombilical. Ces cellules stromales sont capables d'auto-renouvellement et de différenciation en cellules des lignées ostéoblastiques, chondrocytaires et adipocytaires *a minima,* incluant sans limitation, les cellules de l'os, du cartilage, du tissu adipeux et du stroma médullaire, du muscle lisse, des ligaments et des tendons.

On entend par « **cellules souches mésenchymateuses humaines décongelées** », des cellules souches mésenchymateuses isolées à partir des tissus humains et ayant subi le processus de décongélation des cellules souches cryoconservées.

On entend par « **cellules souches mésenchymateuses humaines fraîches** », des cellules souches mésenchymateuses isolées à partir des tissus humains et n'ayant subi aucun processus de congélation-décongélation.

On entend par « **gelée de Wharton** », le tissu conjonctif d'origine mésoblastique extra-embryonnaire, enveloppant les deux artères et la veine ombilicales et protégeant ainsi le cordon ombilical.

On entend par « **sepsis** », une infection générale grave de l'organisme par des germes pathogènes, notamment des bactéries. Le sepsis se définit comme un dysfonctionnement d'organe secondaire à une réponse inappropriée de l'organisme envers une infection. Selon la gravité, l'état septique peut être classé en deux ordres : le sepsis *stricto sensu,* et le choc septique (« The Third International Consensus Definitions for Sepsis and Septic Shock (Sepsis-3) », JAMA. 2016;315(8):801-810). Le sepsis, dans sa définition la plus étroite, associe cliniquement une infection avec un score SOFA (Sequential Organ Failure Assessment Score) ≥ 2 ou une augmentation du score SOFA supérieure ou égale à 2 points s'il existait une dysfonction d'organe avant l'infection. Le choc septique, quant à lui, associe un sepsis avec la nécessité d'utiliser des vasopresseurs qsp PAM [pression artérielle moyenne] ≥ 65 mmHg et/ou une lactatémie > 2 mmol/L (soit 18 mg/dL), et ce, malgré un remplissage vasculaire adéquat.

### DESCRIPTION DÉTAILLÉE

La présente invention a pour objet des cellules souches mésenchymateuses (CSM) pour leur utilisation dans le traitement du sepsis, notamment du choc septique.

Dans un mode de réalisation, les CSM sont des cellules souches animales, de préférence mammifères, de préférence humaines. Dans un mode de réalisation particulier, les CSM sont des cellules souches humaines.

Dans un mode de réalisation, les CSM sont issues de la gelée de Wharton, de la moelle osseuse et/ou du tissu adipeux. Dans un mode de réalisation, les CSM sont issues de la gelée de Wharton. Dans un mode de réalisation, les CSM sont issues de la moelle osseuse. Dans un mode de réalisation, les CSM sont issues du tissu adipeux. Préférentiellement, les CSM sont issues de la gelée de Wharton.

Dans un mode de réalisation, les CSM sont décongelées, *i.e.,* elles ont subi un processus de congélation-décongélation.

Plus particulièrement, la présente invention a donc pour objet des cellules souches mésenchymateuses humaines décongelées issues de la gelée de Wharton (CSM-GW) pour leur utilisation dans le traitement du sepsis, notamment du choc septique.

Dans un mode de réalisation, les CSM décongelées, de préférence les CSM-GW décongelées, présentent des caractéristiques phénotypiques différentes des CSM fraîches, de préférence des CSM-GW fraîches.

Dans un mode de réalisation, les CSM décongelées, de préférence les CSM-GW décongelées, sont caractérisées en ce que :
- au moins 60 % des cellules expriment les antigènes suivants : CD90, CD73, CD105, CD44 ; et
- au moins 80 % des cellules n'expriment aucun des marqueurs suivants : CD34, CD11b, CD19, CD45, HLA-DR ; et
- au moins 10% des cellules expriment le marqueur CD106.

Dans un mode de réalisation, les CSM décongelées, de préférence les CSM-GW décongelées, sont caractérisées en ce que :
- au moins 60 % des cellules expriment les antigènes suivants : CD90, CD73, CD105, CD44 ; et
- au moins 80 % des cellules n'expriment aucun des marqueurs suivants : CD34, CD11b, CD19, CD45, CD144, HLA-DR ; et
- au moins 10% des cellules expriment le marqueur CD106.

Dans un mode de réalisation, les CSM décongelées, de préférence les CSM-GW décongelées, sont caractérisées en ce que le niveau d'expression d'au moins un marqueur sélectionné parmi CD90, CD73, CD105, CD29, CD44, CD146, CD166, HLA-ABC est au moins 5%, au moins 10%, au moins 15%, au moins 20%, au moins 25%, au moins 30%, au moins 35%, au moins 40%, au moins 45%, au moins 50% ou plus inférieur au niveau d'expression du même marqueur dans des CSM fraîches, de préférence dans des CSM-GW fraîches.

Dans un mode de réalisation, les CSM décongelées, de préférence les CSM-GW décongelées, sont caractérisées en ce que le niveau d'expression du marqueur CD90 est au moins 5%, au moins 10%, au moins 15%, au moins 20%, au moins 25%, au moins 30%, au moins 35%, au moins 40%, au moins 45%, au moins 50% ou plus inférieur au niveau d'expression du marqueur CD90 dans des CSM fraîches, de préférence dans des CSM-GW fraîches.

On entend par « **CD90** », la glycoprotéine membranaire Thy-1, dont un exemple est la protéine CD90 humaine dont le numéro d'accession UniProtKB est P04216.

Dans un mode de réalisation, les CSM décongelées, de préférence les CSM-GW décongelées, sont caractérisées en ce que le niveau d'expression du marqueur CD73 est au moins 5%, au moins 10%, au moins 15%, au moins 20%, au moins 25%, au moins 30%, au moins 35%, au moins 40%, au moins 45%, au moins 50% ou plus inférieur au niveau d'expression du marqueur CD73 dans des CSM fraîches, de préférence dans des CSM-GW fraîches.

On entend par « **CD73** », l'enzyme ecto-5'-nucleotidase (ou NT5E), dont un exemple est la protéine CD73 humaine dont le numéro d'accession UniProtKB est P21589.

Dans un mode de réalisation, les CSM décongelées, de préférence les CSM-GW décongelées, sont caractérisées en ce que le niveau d'expression du marqueur CD105 est au moins 5%, au moins 10%, au moins 15%, au moins 20%, au moins 25%, au moins 30%, au moins 35%, au moins 40%, au moins 45%, au moins 50% ou plus inférieur au niveau d'expression du marqueur CD105 dans des CSM fraîches, de préférence dans des CSM-GW fraîches.

On entend par « **CD105** », la glycoprotéine membranaire endogline, dont un exemple est la protéine CD105 humaine dont le numéro d'accession UniProtKB est P17813.

Dans un mode de réalisation, les CSM décongelées, de préférence les CSM-GW décongelées, sont caractérisées en ce que le niveau d'expression du marqueur CD29 est au moins 5%, au moins 10%, au moins 15%, au moins 20%, au moins 25%, au moins 30%, au moins 35%, au moins 40%, au moins 45%, au moins 50% ou plus inférieur au niveau d'expression du marqueur CD29 dans des CSM fraîches, de préférence dans des CSM-GW fraîches.

On entend par « **CD29** », la protéine intégrine β-1, dont un exemple est la protéine CD29 humaine dont le numéro d'accession UniProtKB est P05556.

Dans un mode de réalisation, les CSM décongelées, de préférence les CSM-GW décongelées, sont caractérisées en ce que le niveau d'expression du marqueur CD44 est au moins 5%, au moins 10%, au moins 15%, au moins 20%, au moins 25%, au moins 30%, au moins 35%, au moins 40%, au moins 45%, au moins 50% ou plus inférieur au niveau d'expression du marqueur CD44 dans des CSM fraîches, de préférence dans des CSM-GW fraîches.

On entend par « **CD44** », le récepteur du hyaluronane, dont un exemple est la protéine CD44 humaine dont le numéro d'accession UniProtKB est P16070.

Dans un mode de réalisation, les CSM décongelées, de préférence les CSM-GW décongelées, sont caractérisées en ce que le niveau d'expression du marqueur CD146 est au moins 5%, au moins 10%, au moins 15%, au moins 20%, au moins 25%, au moins 30%, au moins 35%, au moins 40%, au moins 45%, au moins 50% ou plus inférieur au niveau d'expression du marqueur CD146 dans des CSM fraîches, de préférence dans des CSM-GW fraîches.

On entend par « **CD146** », la molécule d'adhésion cellulaire du mélanome (en anglais, *MCAM : Melanoma Cell Adhesion Molecule*), dont un exemple est la protéine CD146 humaine dont le numéro d'accession UniProtKB est P43121.

Dans un mode de réalisation, les CSM décongelées, de préférence les CSM-GW décongelées, sont caractérisées en ce que le niveau d'expression du marqueur CD166 est au moins 5%, au moins 10%, au moins 15%, au moins 20%, au moins 25%, au moins 30%, au moins 35%, au moins 40%, au moins 45%, au moins 50% ou plus inférieur au niveau d'expression du marqueur CD166 dans des CSM fraîches, de préférence dans des CSM-GW fraîches.

On entend par « **CD166** », la molécule d'adhésion cellulaire des leucocytes activés (en anglais, *ALCAM: Activated Leukocyte Cell Adhesion Molecule*), dont un exemple est la protéine CD166 humaine dont le numéro d'accession UniProtKB est Q13740.

Dans un mode de réalisation, les CSM décongelées, de préférence les CSM-GW décongelées, sont caractérisées en ce que le niveau d'expression du marqueur HLA-ABC est au moins 5%, au moins 10%, au moins 15%, au moins 20%, au moins 25%, au moins 30%, au moins 35%, au moins 40%, au moins 45%, au moins 50% ou plus inférieur au niveau d'expression du marqueur HLA-ABC dans des CSM fraîches, de préférence dans des CSM-GW fraîches.

On entend par « **HLA-ABC** », les récepteurs de surface du complexe majeur d'histocompatibilité de classe I, dont un exemple est l'HLA-ABC humaine dont le numéro d'accession UniProtKB est 019689.

Dans un mode de réalisation, les CSM décongelées, de préférence les CSM-GW décongelées, sont caractérisées en ce que le niveau d'expression d'au moins un marqueur sélectionné parmi CD13 et Sox-2 est au moins 5%, au moins 10%, au moins 15%, au moins 20%, au moins 25%, au moins 30%, au moins 35%, au moins 40%, au moins 45%, au moins 50% ou plus supérieur au niveau d'expression du même marqueur dans des CSM fraîches, de préférence dans des CSM-GW fraîches.

Dans un mode de réalisation, les CSM décongelées, de préférence les CSM-GW décongelées, sont caractérisées en ce que le niveau d'expression du marqueur CD13 est au moins 5%, au moins 10%, au moins 15%, au moins 20%, au moins 25%, au moins 30%, au moins 35%, au moins 40%, au moins 45%, au moins 50% ou plus supérieur au niveau d'expression du marqueur CD13 dans des CSM fraîches, de préférence dans des CSM-GW fraîches.

On entend par « **CD13** », l'alanyl aminopeptidase de membrane, dont un exemple est la protéine CD13 humaine dont le numéro d'accession UniProtKB est P15144.

Dans un mode de réalisation, les CSM décongelées, de préférence les CSM-GW décongelées, sont caractérisées en ce que le niveau d'expression du marqueur Sox-2 est au moins 5%, au moins 10%, au moins 15%, au moins 20%, au moins 25%, au moins 30%, au moins 35%, au moins 40%, au moins 45%, au moins 50% ou plus supérieur au niveau d'expression du marqueur Sox-2 dans des CSM fraîches, de préférence dans des CSM-GW fraîches.

On entend par « **Sox-2** », le facteur de transcription SRY-box 2, dont un exemple est la protéine Sox-2 humaine dont le numéro d'accession UniProtKB est P48431.

Dans un mode de réalisation, les CSM décongelées, de préférence les CSM-GW décongelées, après au moins un passage en sous-culture, sont caractérisées en ce que le niveau d'expression d'au moins un marqueur sélectionné parmi CD44 et SSEA-4 est au moins 5%, au moins 10%, au moins 15%, au moins 20%, au moins 25%, au moins 30%, au moins 35%, au moins 40%, au moins 45%, au moins 50% ou plus inférieur au niveau d'expression du même marqueur dans des CSM fraîches, de préférence dans des CSM-GW fraîches.

Dans un mode de réalisation, les CSM décongelées, de préférence les CSM-GW décongelées, après au moins un passage en sous-culture, sont caractérisées en ce que le niveau d'expression du marqueur CD44 est au moins 5%, au moins 10%, au moins 15%, au moins 20%, au moins 25%, au moins 30%, au moins 35%, au moins 40%, au moins 45%, au moins 50% ou plus inférieur au niveau d'expression du marqueur CD44 dans des CSM fraîches, de préférence dans des CSM-GW fraîches.

Dans un mode de réalisation, les CSM décongelées, de préférence les CSM-GW décongelées, après au moins un passage en sous-culture, sont caractérisées en ce que le niveau d'expression du marqueur SSEA-4 est au moins 5%, au moins 10%, au moins 15%, au moins 20%, au moins 25%, au moins 30%, au moins 35%, au moins 40%, au moins 45%, au moins 50% ou plus inférieur au niveau d'expression du marqueur SSEA4 dans des CSM fraîches, de préférence dans des CSM-GW fraîches.

On entend par « **SSEA-4** », le ganglioside membranaire constitué d'un glycosphingolipide comprenant un résidu d'acide sialique terminale (en anglais, *Stage-Specific Embryonic Antigen 4).*

Dans un mode de réalisation, les CSM décongelées, de préférence les CSM-GW décongelées, après au moins un passage en sous-culture, sont caractérisées en ce que le niveau d'expression d'au moins un marqueur sélectionné parmi CD90, CD166 et HLA-ABC est au moins 5%, au moins 10%, au moins 15%, au moins 20%, au moins 25%, au moins 30%, au moins 35%, au moins 40%, au moins 45%, au moins 50% ou plus supérieur au niveau d'expression du même marqueur dans des CSM fraîches, de préférence dans des CSM-GW fraîches.

Dans un mode de réalisation, les CSM décongelées, de préférence les CSM-GW décongelées, après au moins un passage en sous-culture, sont caractérisées en ce que le niveau d'expression du marqueur CD90 est au moins 5%, au moins 10%, au moins 15%, au moins 20%, au moins 25%, au moins 30%, au moins 35%, au moins 40%, au moins 45%, au moins 50% ou plus supérieur au niveau d'expression du marqueur CD90 dans des CSM fraîches, de préférence dans des CSM-GW fraîches.

Dans un mode de réalisation, les CSM décongelées, de préférence les CSM-GW décongelées, après au moins un passage en sous-culture, sont caractérisées en ce que le niveau d'expression du marqueur CD166 est au moins 5%, au moins 10%, au moins 15%, au moins 20%, au moins 25%, au moins 30%, au moins 35%, au moins 40%, au moins 45%, au moins 50% ou plus supérieur au niveau d'expression du marqueur CD166 dans des CSM fraîches, de préférence dans des CSM-GW fraîches.

Dans un mode de réalisation, les CSM décongelées, de préférence les CSM-GW décongelées, après au moins un passage en sous-culture, sont caractérisées en ce que le niveau d'expression du marqueur HLA-ABC est au moins 5%, au moins 10%, au moins 15%, au moins 20%, au moins 25%, au moins 30%, au moins 35%, au moins 40%, au moins 45%, au moins 50% ou plus supérieur au niveau d'expression du marqueur HLA-ABC dans des CSM fraîches, de préférence dans des CSM-GW fraîches.

Dans un mode de réalisation, le niveau d'expression des marqueurs est mesuré par des méthodes bien connues de l'homme du métier. Dans un mode de réalisation, le niveau d'expression des marqueurs est mesuré par cytométrie de flux. Dans un mode de réalisation, le niveau d'expression des marqueurs est mesuré par intensité moyenne de fluorescence (en anglais, *MFI : Mean Fluorescence Intensity*).

Dans un mode de réalisation, les CSM décongelées, de préférence les CSM-GW décongelées, sont caractérisées par une expression protéique différentielle par rapport aux CSM fraîches, de préférence aux CSM-GW fraîches.

Dans un mode de réalisation, les CSM décongelées, de préférence les CSM-GW décongelées, expriment au moins une protéine sélectionnée parmi le groupe comprenant ou consistant en ACTB (cytoplasmic actin 1), ANXA1 (annexin A1), CAPZB (F-actin-capping protein subunit beta), LASP1 (LIM and SH3 domain protein 1), PRDX2 (peroxiredoxin-2), PRDX3 (mitochondrial thioredoxin-dependent peroxide reductase), PSA3 (proteasome subunit alpha type-3), RS12 (40S ribosomal protein S12) et SYWC (cytoplasmic tryptophan-tRNA ligase).

Dans un mode de réalisation, les CSM fraîches, de préférence les CSM-GW fraîches, n'expriment pas ou substantiellement pas au moins une protéine sélectionnée parmi le groupe comprenant ou consistant en ACTB (cytoplasmic actin 1), ANXA1 (annexin A1), CAPZB (F-actin-capping protein subunit beta), LASP1 (LIM and SH3 domain protein 1), PRDX2 (peroxiredoxin-2), PRDX3 (mitochondrial thioredoxin-dependent peroxide reductase), PSA3 (proteasome subunit alpha type-3), RS12 (40S ribosomal protein S12) et SYWC (cytoplasmic tryptophan-tRNA ligase).

Dans un mode de réalisation, les CSM décongelées, de préférence les CSM-GW décongelées, expriment au moins 1.1 fois, 1.2 fois, 1.3 fois, 1.4 fois, 1.5 fois, 1.6 fois, 1.7 fois, 1.8 fois, 1.9 fois, 2 fois, 2.5 fois, 3.0 fois, 3.5 fois, 4.0 fois, 4.5 fois, 5.0 fois, 7.5 fois, 10.0 fois, 15.0 fois, 20.0 fois, 30.0 fois, 40.0 fois, 50.0 fois plus au moins une protéine sélectionnée parmi le groupe comprenant ou consistant en ACTB (cytoplasmic actin 1), ANXA1 (annexin A1), CAPZB (F-actin-capping protein subunit beta), LASP1 (LIM and SH3 domain protein 1), PRDX2 (peroxiredoxin-2), PRDX3 (mitochondrial thioredoxin-dependent peroxide reductase), PSA3 (proteasome subunit alpha type-3), RS12 (40S ribosomal protein S12) et SYWC (cytoplasmic tryptophan-tRNA ligase), que les CSM fraîches, de préférence que les CSM-GW fraîches.

On entend par « **ACTB** », la protéine d'actine 1 cytoplasmique (en anglais, *cytoplasmic actin 1*), dont un exemple est l'ACTB humaine dont le numéro d'accession UniProtKB est P60709.

On entend par « **ANXA1** », la protéine d'annexine 1 (en anglais, *annexin A1*), dont un exemple est l'ANXA1 humaine dont le numéro d'accession UniProtKB est P04083.

On entend par « **CAPZB** », la sous-unité β de la protéine de capping de l'actine F (en anglais, *F-actin-capping protein subunit beta),* dont un exemple est la CAPZB humaine dont le numéro d'accession UniProtKB est P47756.

On entend par « **LASP1** », la protéine 1 de domaine LIM et SH3 (en anglais, *LIM and SH3 domain protein 1)*, dont un exemple est la LASP1 humaine dont le numéro d'accession UniProtKB est Q14847.

On entend par « **PRDX2** », la peroxirédoxine 2 (en anglais, *peroxiredoxin-2*)*,* dont un exemple est la PRDX2 humaine dont le numéro d'accession UniProtKB est P32119.

On entend par « **PRDX3** », la peroxirédoxine 3 (en anglais, *mitochondrial thioredoxin-dependent peroxide reductase*), dont un exemple est la PRDX3 humaine dont le numéro d'accession UniProtKB est P30048.

On entend par « **PSA3** », la sous-unité α de type 3 du protéasome (en anglais, *proteasome subunit alpha type-3),* dont un exemple est la PSA3 humaine dont le numéro d'accession UniProtKB est P25788.

On entend par « **RS12** », la protéine S12 de la sous-unité 40S du ribosome (en anglais, *40S ribosomal protein S12*), dont un exemple est la RS12 humaine dont le numéro d'accession UniProtKB est P25398.

On entend par « **SYWC** », la tryptophanyl-ARNt synthétase cytoplasmique (en anglais, *cytoplasmic tryptophan-tRNA ligase*), dont un exemple est la SYWC humaine dont le numéro d'accession UniProtKB est P23381.

Dans un mode de réalisation, les CSM décongelées, de préférence les CSM-GW décongelées, n'expriment pas ou substantiellement pas au moins une protéine sélectionnée parmi le groupe comprenant ou consistant en ACTS (skeletal muscle alpha actin), AL1B1 (mitochondrial aldehyde dehydrogenase X), ANX10 (annexin A10), GBB1 (guanine nucleotide-binding protein G(I)/G(S)/G(T) subunit beta-1), GBB2 (guanine nucleotide-binding protein G(I)/G(S)/G(T) subunit beta-2), GPRIN1 (G protein-regulated inducer of neurite outgrowth 1), DTNA (dystrobrevin alpha), MIPO1 (mirror-image polydactyly gene 1 protein), PSB3 (proteasome subunit beta type-3) et PSDE (26S proteasome non-ATPase regulatory subunit 14).

Dans un mode de réalisation, les CSM fraîches, de préférence les CSM-GW fraîches, expriment au moins une protéine sélectionnée parmi le groupe comprenant ou consistant en ACTS (skeletal muscle alpha actin), AL1B1 (mitochondrial aldehyde dehydrogenase X), ANX10 (annexin A10), GBB1 (guanine nucleotide-binding protein G(I)/G(S)/G(T) subunit beta-1), GBB2 (guanine nucleotide-binding protein G(I)/G(S)/G(T) subunit beta-2), GPRIN1 (G protein-regulated inducer of neurite outgrowth 1), DTNA (dystrobrevin alpha), MIPO1 (mirror-image polydactyly gene 1 protein), PSB3 (proteasome subunit beta type-3) et PSDE (26S proteasome non-ATPase regulatory subunit 14).

Dans un mode de réalisation, les CSM décongelées, de préférence les CSM-GW décongelées, expriment au moins 1.1 fois, 1.2 fois, 1.3 fois, 1.4 fois, 1.5 fois, 1.6 fois, 1.7 fois, 1.8 fois, 1.9 fois, 2 fois, 2.5 fois, 3.0 fois, 3.5 fois, 4.0 fois, 4.5 fois, 5.0 fois, 7.5 fois, 10.0 fois, 15.0 fois, 20.0 fois, 30.0 fois, 40.0 fois, 50.0 fois moins au moins une protéine sélectionnée parmi le groupe comprenant ou consistant en ACTS (skeletal muscle alpha actin), AL1B1 (mitochondrial aldehyde dehydrogenase X), ANX10 (annexin A10), GBB1 (guanine nucleotide-binding protein G(I)/G(S)/G(T) subunit beta-1), GBB2 (guanine nucleotide-binding protein G(I)/G(S)/G(T) subunit beta-2), GPRIN1 (G protein-regulated inducer of neurite outgrowth 1), DTNA (dystrobrevin alpha), MIPO1 (mirror-image polydactyly gene 1 protein), PSB3 (proteasome subunit beta type-3) et PSDE (26S proteasome non-ATPase regulatory subunit 14), que les CSM fraîches, de préférence que les CSM-GW fraîches.

On entend par « **ACTS** », la protéine d'actine α du muscle squelettique (en anglais, *skeletal muscle alpha actin*), dont un exemple est l'ACTS humaine dont le numéro d'accession UniProtKB est P68133.

On entend par « **ALIBI** », l'aldéhyde déshydrogénase X mitochondriale (en anglais, *mitochondrial aldehyde dehydrogenase X*)*,* dont un exemple est l'AL1B1 humaine dont le numéro d'accession UniProtKB est P30837.

On entend par « **ANX10** », la protéine d'annexine A10 (en anglais, *annexin A10*), dont un exemple est l'ANX10 humaine dont le numéro d'accession UniProtKB est Q9UJ72.

On entend par « **GBB1** », la sous-unité β1 de la protéine de liaison des guanines G(I)/G(S)/G(T) (en anglais, *guanine nucleotide-binding protein G(I)*/*G(S)*/*G(T) subunit beta-1*), dont un exemple est la GBB1 humaine dont le numéro d'accession UniProtKB est P62873.

On entend par « **GBB2** », la sous-unité β2 de la protéine de liaison des guanines G(I)/G(S)/G(T) (en anglais, *guanine nucleotide-binding protein G(I)*/*G(S)*/*G(T) subunit beta-2*), dont un exemple est la GBB2 humaine dont le numéro d'accession UniProtKB est P62879.

On entend par « **GPRIN1** », l'inducteur régulé par la protéine G de croissance des neurites 1 (en anglais, *G protein-regulated inducer of neurite outgrowth 1*), dont un exemple est la GPRIN1 humaine dont le numéro d'accession UniProtKB est Q7Z2K8.

On entend par « **DTNA** », la dystrobrévine α (en anglais, *dystrobrevin alpha*), dont un exemple est la DTNA humaine dont le numéro d'accession UniProtKB est Q9Y4J8.

On entend par « **MIPO1** », la protéine du gène 1 de la polydactylie en miroir (en anglais, *mirror-image polydactyly gene 1 protein),* dont un exemple est la MIPO1 humaine dont le numéro d'accession UniProtKB est Q8TD10.

On entend par « **PSB3** », la sous-unité β de type 3 du protéasome (en anglais, *proteasome subunit beta type-3),* dont un exemple est la PSB3 humaine dont le numéro d'accession UniProtKB est P49720.

On entend par « **PSDE** », la sous-unité régulatoire 14 non-ATPase du protéasome 26S (en anglais, *26S proteasome non-ATPase regulatory subunit 14),* dont un exemple est la PSDE humaine dont le numéro d'accession UniProtKB est O00487.

Dans un mode de réalisation, les CSM décongelées, de préférence les CSM-GW décongelées, après au moins 1 passage en sous-culture, expriment au moins 1.1 fois, 1.2 fois, 1.3 fois, 1.4 fois, 1.5 fois, 1.6 fois, 1.7 fois, 1.8 fois, 1.9 fois, 2 fois, 2.5 fois, 3.0 fois, 3.5 fois, 4.0 fois, 4.5 fois, 5.0 fois, 7.5 fois, 10.0 fois, 15.0 fois, 20.0 fois, 30.0 fois, 40.0 fois, 50.0 fois plus au moins une protéine sélectionnée parmi le groupe comprenant ou consistant en AMPM2 (methionine aminopeptidase 2), DJB11 (dnaJ homolog subfamily B member 11), K1C18 (keratin, type I cytoskeletal 18), K1C19 (keratin, type I cytoskeletal 19), K2C8 (keratin type II cytoskeletal 8), LYSC (lysozyme C), PDIA3 (protein disulfide-isomerase A3), TCTP (translationally-controlled tumor protein) et TGM2 (protein-glutamine gamma-glutamyltransferase 2) que les CSM fraîches, de préférence que les CSM-GW fraîches.

On entend par « **AMPM2** », la méthionyl aminopeptidase 2 (en anglais, *methionine aminopeptidase 2),* dont un exemple est l'AMPM2 humaine dont le numéro d'accession UniProtKB est P50579.

On entend par « **DJB11** », le membre 11 de la sous-famille B des homologues de HSP40 (en anglais, *dnaJ homolog subfamily B member 11*)*,* dont un exemple est la DJB11 humaine dont le numéro d'accession UniProtKB est Q9UBS4.

On entend par « **K1C18** », la cytokératine 18 de type I acide (en anglais, *keratin, type I cytoskeletal 18),* dont un exemple est la K1C18 humaine dont le numéro d'accession UniProtKB est P05783.

On entend par « **K1C19** », la cytokératine 19 de type I acide (en anglais, *keratin, type I cytoskeletal 19),* dont un exemple est la K1C19 humaine dont le numéro d'accession UniProtKB est P08727.

On entend par « **K2C8** », la cytokératine 8 de type II basique (en anglais, *keratin type II cytoskeletal 8),* dont un exemple est la K2C8 humaine dont le numéro d'accession UniProtKB est P05787.

On entend par « **LYSC** », le lysozyme C (en anglais, *lysozyme C*), dont un exemple est la LYSC humaine dont le numéro d'accession UniProtKB est P61626.

On entend par « **PDIA3** », l'enzyme disulfure isomérase A3 (en anglais, *protein disulfide-isomerase A3),* dont un exemple est la PDIA3 humaine dont le numéro d'accession UniProtKB est P30101.

On entend par « **TCTP** », la protéine tumorale contrôlée traductionnelle (en anglais, *translationally-controlled tumor protein*), dont un exemple est la TCTP humaine dont le numéro d'accession UniProtKB est P13693.

On entend par « **TGM2** », l'enzyme γ-glutamyltranspeptidase 2 (en anglais, *protein-glutamine gamma-glutamyltransferase* 2), dont un exemple est la TGM2 humaine dont le numéro d'accession UniProtKB est P21980.

Dans un mode de réalisation, les CSM décongelées, de préférence les CSM-GW décongelées, après au moins 1 passage en sous-culture, expriment au moins 1.1 fois, 1.2 fois, 1.3 fois, 1.4 fois, 1.5 fois, 1.6 fois, 1.7 fois, 1.8 fois, 1.9 fois, 2 fois, 2.5 fois, 3.0 fois, 3.5 fois, 4.0 fois, 4.5 fois, 5.0 fois, 7.5 fois, 10.0 fois, 15.0 fois, 20.0 fois, 30.0 fois, 40.0 fois, 50.0 fois moins au moins une protéine sélectionnée parmi le groupe comprenant ou consistant en GSTP1 (glutathione S-transferase P), HSP7C (heat shock cognate 71 kDa protein), HSPB1 (heat shock protein beta-1), LEG1 (galectin-1), S10AB (protein S100-A11) et UBE2N (ubiquitin-conjugating enzyme E2 N), que les CSM fraîches, de préférence que les CSM-GW fraîches.

On entend par « **GSTP1** », l'enzyme glutathion S-transférase P (en anglais, *glutathione S-transferase P),* dont un exemple est la GSTP1 humaine dont le numéro d'accession UniProtKB est P09211.

On entend par « **HSP7C** », la protéine de choc thermique 73 (en anglais, *heat shock cognate 71 kDa protein),* dont un exemple est la HSP7C humaine dont le numéro d'accession UniProtKB est P11142.

On entend par « **HSPB1** », la protéine de choc thermique 27 (en anglais, *heat shock protein beta-1*), dont un exemple est la HSPB1 humaine dont le numéro d'accession UniProtKB est P04792.

On entend par « **LEG1** », la galectine 1 (en anglais, *galectin-1*), dont un exemple est la LEG1 humaine dont le numéro d'accession UniProtKB est P09382.

On entend par « **S10AB** », la protéine S100-A11 (en anglais, *protein S100-A11*), dont un exemple est la S10AB humaine dont le numéro d'accession UniProtKB est P31949.

On entend par « **UBE2N** », l'enzyme de conjugaison d'ubiquitine E2 N (en anglais, *ubiquitin-conjugating enzyme E2 N),* dont un exemple est l'UBE2N humaine dont le numéro d'accession UniProtKB est P61088.

Dans un mode de réalisation, l'expression protéique est mesurée par des méthodes bien connues de l'homme du métier. Dans un mode de réalisation, l'expression protéique est mesurée par spectrométrie de masse.

Dans un mode de réalisation, les CSM décongelées, de préférence les CSM-GW décongelées, en condition *in vitro* et/ou en condition non-inflammatoire, sécrètent au moins un facteur de croissance sélectionné parmi BMP-7, IGFBP-1, insuline, FGF-7, NT-4 et VEGF-D.

Dans un mode de réalisation, les CSM décongelées, de préférence les CSM-GW décongelées, en condition *in vitro* et/ou en condition non-inflammatoire, sécrètent au moins 1.1 fois, 1.2 fois, 1.3 fois, 1.4 fois, 1.5 fois, 1.6 fois, 1.7 fois, 1.8 fois, 1.9 fois, 2 fois, 2.5 fois, 3.0 fois, 3.5 fois, 4.0 fois, 4.5 fois, 5.0 fois, 7.5 fois, 10.0 fois, 15.0 fois, 20.0 fois, 30.0 fois, 40.0 fois, 50.0 fois plus au moins un facteur de croissance sélectionné parmi BMP-7, IGFBP-1, insuline, FGF-7, NT-4 et VEGF-D, que les CSM fraîches, de préférence que les CSM-GW fraîches, en condition *in vitro* et/ou en condition non-inflammatoire.

Dans un mode de réalisation, les CSM décongelées, de préférence les CSM-GW décongelées, en condition *in vitro* et/ou en condition non-inflammatoire, sécrètent au moins 50 pg/mL, au moins 100 pg/mL, au moins 150 pg/mL, au moins 200 pg/mL, au moins 250 pg/mL, au moins 300 pg/mL, au moins 350 pg/mL, au moins 400 pg/mL, au moins 450 pg/mL, au moins 500 pg/mL ou plus de BMP-7.

Dans un mode de réalisation, les CSM décongelées, de préférence les CSM-GW décongelées, en condition *in vitro* et/ou en condition non-inflammatoire, sécrètent au moins 20 pg/mL, au moins 25 pg/mL, au moins 30 pg/mL, au moins 35 pg/mL, au moins 40 pg/mL, au moins 45 pg/mL, au moins 50 pg/mL, au moins 55 pg/mL, au moins 60 pg/mL, au moins 65 pg/mL ou plus d'IGFBP-1.

Dans un mode de réalisation, les CSM décongelées, de préférence les CSM-GW décongelées, en condition *in vitro* et/ou en condition non-inflammatoire, sécrètent au moins 50 pg/mL, au moins 75 pg/mL, au moins 100 pg/mL, au moins 125 pg/mL, au moins 150 pg/mL, au moins 175 pg/mL, au moins 200 pg/mL, au moins 225 pg/mL, au moins 250 pg/mL, au moins 275 pg/mL ou plus d'insuline.

Dans un mode de réalisation, les CSM décongelées, de préférence les CSM-GW décongelées, en condition *in vitro* et/ou en condition non-inflammatoire, sécrètent au moins 10 pg/mL, au moins 15 pg/mL, au moins 20 pg/mL, au moins 25 pg/mL, au moins 30 pg/mL, au moins 35 pg/mL, au moins 40 pg/mL, au moins 45 pg/mL, au moins 50 pg/mL, au moins 55 pg/mL, au moins 60 pg/mL, au moins 65 pg/mL ou plus de FGF-7.

Dans un mode de réalisation, les CSM décongelées, de préférence les CSM-GW décongelées, en condition *in vitro* et/ou en condition non-inflammatoire, sécrètent au moins 45 pg/mL, au moins 50 pg/mL, au moins 55 pg/mL, au moins 60 pg/mL, au moins 65 pg/mL, au moins 70 pg/mL, au moins 75 pg/mL, au moins 80 pg/mL, au moins 85 pg/mL, au moins 90 pg/mL ou plus de NT-4.

Dans un mode de réalisation, les CSM décongelées, de préférence les CSM-GW décongelées, en condition *in vitro* et/ou en condition non-inflammatoire, sécrètent au moins 5 pg/mL, au moins 10 pg/mL, au moins 15 pg/mL, au moins 20 pg/mL, au moins 25 pg/mL, au moins 30 pg/mL, au moins 35 pg/mL, au moins 40 pg/mL, au moins 45 pg/mL, au moins 50 pg/mL ou plus de VEGF-D.

Dans un mode de réalisation, les CSM décongelées, de préférence les CSM-GW décongelées, en condition *in vivo* et/ou en condition inflammatoire, sécrètent au moins un facteur de croissance sélectionné parmi BMP-7 et TGFβ3.

Dans un mode de réalisation, les CSM décongelées, de préférence les CSM-GW décongelées, en condition *in vivo* et/ou en condition inflammatoire, sécrètent au moins 1.1 fois, 1.2 fois, 1.3 fois, 1.4 fois, 1.5 fois, 1.6 fois, 1.7 fois, 1.8 fois, 1.9 fois, 2 fois, 2.5 fois, 3.0 fois, 3.5 fois, 4.0 fois, 4.5 fois, 5.0 fois, 7.5 fois, 10.0 fois, 15.0 fois, 20.0 fois, 30.0 fois, 40.0 fois, 50.0 fois plus d'au moins un facteur de croissance sélectionné parmi BMP-7 et TGFβ3, que les CSM fraîches, de préférence que les CSM-GW fraîches, en condition *in vivo* et/ou en condition inflammatoire.

Dans un mode de réalisation, les CSM décongelées, de préférence les CSM-GW décongelées, en condition *in vivo* et/ou en condition inflammatoire, sécrètent au moins 200 pg/mL, au moins 250 pg/mL, au moins 300 pg/mL, au moins 350 pg/mL, au moins 400 pg/mL, au moins 450 pg/mL, au moins 500 pg/mL, au moins 550 pg/mL, au moins 600 pg/mL, au moins 650 pg/mL, au moins 700 pg/mL, au moins 750 pg/mL ou plus de BMP-7.

Dans un mode de réalisation, les CSM décongelées, de préférence les CSM-GW décongelées, en condition *in vivo* et/ou en condition inflammatoire, sécrètent au moins 20 pg/mL, au moins 25 pg/mL, au moins 30 pg/mL, au moins 35 pg/mL, au moins 40 pg/mL, au moins 45 pg/mL, au moins 50 pg/mL, au moins 55 pg/mL, au moins 60 pg/mL, au moins 65 pg/mL ou plus TGFβ3.

Dans un mode de réalisation, les CSM décongelées, de préférence les CSM-GW décongelées, en condition *in vivo* et/ou en condition inflammatoire, ne sécrètent pas ou substantiellement pas d'IGFBP-1.

Dans un mode de réalisation, les CSM décongelées, de préférence les CSM-GW décongelées, en condition *in vivo* et/ou en condition inflammatoire, sécrètent au moins 1.1 fois, 1.2 fois, 1.3 fois, 1.4 fois, 1.5 fois, 1.6 fois, 1.7 fois, 1.8 fois, 1.9 fois, 2 fois, 2.5 fois, 3.0 fois, 3.5 fois, 4.0 fois, 4.5 fois, 5.0 fois, 7.5 fois, 10.0 fois, 15.0 fois, 20.0 fois, 30.0 fois, 40.0 fois, 50.0 fois moins d'IGFBP-1, que les CSM fraîches, de préférence que les CSM-GW fraîches, en condition *in vivo* et/ou en condition inflammatoire.

Dans un mode de réalisation, les CSM décongelées, de préférence les CSM-GW décongelées, en condition *in vivo* et/ou en condition inflammatoire, sécrètent moins de 20 pg/mL, au moins 15 pg/mL, au moins 10 pg/mL, au moins 5 pg/mL, au moins 4 pg/mL, au moins 3 pg/mL, au moins 2 pg/mL, au moins 1 pg/mL ou moins IGFBP-1.

On entend par « **BMP-7** », la protéine morphogénétique osseuse 7 (en anglais, *Bone Morphogenetic Protein* 7). Un exemple de BMP-7 correspond à la protéine BMP-7 humaine dont le numéro d'accession UniProtKB est P18075.

On entend par « **IGFBP-1** », la protéine de liaison du facteur de croissance 1 ressemblant à l'insuline (en anglais, *Insulin-like Growth Factor-Binding Protein 1*)*.* Un exemple de IGFBP-1 correspond à la protéine IGFBP-1 humaine dont le numéro d'accession UniProtKB est P08833.

On entend par « **insuline** », l'hormone protéique favorisant l'absorption du glucose sanguin. Un exemple d'insuline correspond à l'insuline humaine dont le numéro d'accession UniProtKB est P01308.

On entend par « **FGF-7** », le facteur de croissance des fibroblastes 7 (en anglais, *Fibroblast Growth Factor 7*). Un exemple de FGF-7 correspond à la protéine FGF-7 humaine dont le numéro d'accession UniProtKB est P21781.

On entend par « **NT-4** », la neurotrophine 4. Un exemple de NT-4 correspond à la protéine NT-4 humaine dont le numéro d'accession UniProtKB est P34130.

On entend par « **VEGF-D** », le facteur de croissance de l'endothélium vasculaire D (en anglais, *Vascular Endothelial Growth Factor D*)*.* Un exemple de VEGF-D correspond à la protéine VEGF-D humaine dont le numéro d'accession UniProtKB est O43915.

On entend par « **TGFβ3** », le facteur de croissance transformant β3 (en anglais, *Transforming Growth Factor β3*). Un exemple de TGFβ3 correspond à la protéine TGFβ3 humaine dont le numéro d'accession UniProtKB est P10600.

On entend par « **condition *in vitro*** », une condition dans laquelle les CSM décongelées, de préférence les CSM-GW décongelées sont en dehors d'un organisme vivant, de préférence en dehors du patient.

On entend par « **condition *in vivo*** », une condition dans laquelle les CSM décongelées, de préférence les CSM-GW décongelées sont à l'intérieur d'un organisme vivant, de préférence à l'intérieur du patient, *i.e.,* après leur administration au patient.

On entend par « **condition non-inflammatoire** », une condition dans laquelle les CSM décongelées, de préférence les CSM-GW décongelées ne sont pas stimulées, *in vitro* ou *in vivo,* par des cytokines inflammatoires, incluant mais n'étant pas limitées à, TNF-α et IFN-γ. Particulièrement, une condition non-inflammatoire peut se définir comme une condition *in vivo,* de préférence à l'intérieur du patient, ledit patient étant sain ou substantiellement sain, *i.e.,* ledit patient n'est pas affecté par un sepsis.

On entend par « **condition inflammatoire** », une condition dans laquelle les CSM décongelées, de préférence les CSM-GW décongelées sont stimulées, *in vitro* ou *in vivo,* par des cytokines inflammatoires, incluant mais n'étant pas limitées à, TNF-α et IFN-γ. Particulièrement, une condition inflammatoire peut se définir comme une condition *in vivo,* de préférence à l'intérieur du patient, ledit patient étant affecté par un sepsis.

Dans un mode de réalisation, les CSM décongelées, de préférence les CSM-GW décongelées, sécrètent du VEGF.

Dans un mode de réalisation, les CSM décongelées, de préférence les CSM-GW décongelées, sécrètent au moins 1.1 fois, 1.2 fois, 1.3 fois, 1.4 fois, 1.5 fois, 1.6 fois, 1.7 fois, 1.8 fois, 1.9 fois, 2 fois, 2.5 fois, 3.0 fois, 3.5 fois, 4.0 fois, 4.5 fois, 5.0 fois, 7.5 fois, 10.0 fois, 15.0 fois, 20.0 fois, 30.0 fois, 40.0 fois, 50.0 fois plus de VEGF que les CSM fraîches, de préférence que les CSM-GW fraîches.

Dans un mode de réalisation, les CSM décongelées, de préférence les CSM-GW décongelées, sécrètent au moins 500 pg/mL, au moins 600 pg/mL, au moins 700 pg/mL, au moins 800 pg/mL, au moins 900 pg/mL, au moins 1 ng/mL, au moins 1.25 ng/mL, au moins 1.5 ng/mL, au moins 1.75 ng/mL, au moins 2 ng/mL ou plus de VEGF.

Dans un mode de réalisation, les CSM décongelées, de préférence les CSM-GW décongelées, ne sécrètent pas ou substantiellement pas de VEGF.

Dans un mode de réalisation, les CSM décongelées, de préférence les CSM-GW décongelées, sécrètent au moins 1.1 fois, 1.2 fois, 1.3 fois, 1.4 fois, 1.5 fois, 1.6 fois, 1.7 fois, 1.8 fois, 1.9 fois, 2 fois, 2.5 fois, 3.0 fois, 3.5 fois, 4.0 fois, 4.5 fois, 5.0 fois, 7.5 fois, 10.0 fois, 15.0 fois, 20.0 fois, 30.0 fois, 40.0 fois, 50.0 fois moins de VEGF, que les CSM fraîches, de préférence que les CSM-GW fraîches.

Dans un mode de réalisation, les CSM décongelées, de préférence les CSM-GW décongelées, sécrètent moins de 500 pg/mL, au moins 400 pg/mL, au moins 300 pg/mL, au moins 250 pg/mL, au moins 200 pg/mL, au moins 150 pg/mL, au moins 100 pg/mL, au moins 50 pg/mL ou moins de VEGF.

On entend par « **VEGF** », le facteur de croissance de l'endothélium vasculaire (en anglais, *Vascular Endothelial Growth Factor*). Un exemple de VEGF correspond à la protéine VEGF humaine dont le numéro d'accession UniProtKB est P15692.

Dans un mode de réalisation, les CSM décongelées, de préférence les CSM-GW décongelées, en condition *in vivo* et/ou en condition inflammatoire, induisent la sécrétion de VEGF.

Plusieurs cellules peuvent sécréter du VEGF. Des exemples de telles cellules incluent, sans y être limitées, les macrophages, les monocytes, les cellules endothéliales, les myofibroblastes, les chondrocytes, et les cellules hématopoïétiques.

Dans un mode de réalisation, les CSM décongelées, de préférence les CSM-GW décongelées, induisent une augmentation de la concentration sérique de VEGF d'au moins 5%, 10%, 15%, 20%, 25%, 30%, 35%, 40%, 45%, 50% ou plus, par rapport aux CSM fraîches, de préférence par rapport aux CSM-GW fraîches.

Dans un mode de réalisation, les CSM décongelées, de préférence les CSM-GW décongelées, induisent une augmentation de la concentration plasmatique de VEGF d'au moins 5%, 10%, 15%, 20%, 25%, 30%, 35%, 40%, 45%, 50% ou plus, par rapport aux CSM fraîches, de préférence par rapport aux CSM-GW fraîches.

Dans un mode de réalisation, les CSM, de préférence les CSM-GW, sont conservées à très basse température. Dans un mode de réalisation, les CSM, de préférence les CSM-GW, sont conservées à environ -20°C, de préférence à environ -80°C, de préférence entre -150°C et -196°C. Dans un mode de réalisation, les CSM, de préférence les CSM-GW, sont conservées en présence d'une solution cryoprotectrice. Les solutions cryoprotectrices de cellules sont bien connues de l'homme du métier. De telles solution comprennent, e.g., Dans un mode de réalisation, la solution cryoprotectrice comprend au moins un agent cryoprotecteur sélectionné parmi le dimethylsulfoxide (DMSO), le glycérol, l'éthylène glycol, le propylène glycol, le formamide, le butène diol et un mélange de ceux-ci.

Dans un mode de réalisation, les CSM, de préférence les CSM-GW, sont décongelées selon un protocole de décongélation conventionnel. À titre d'exemple, les CSM, de préférence les CSM-GW, sont décongelées au bain-marie à 37°C puis lavées en présence d'une solution de lavage comprenant du NaCl, de l'albumine et du citrate dextrose formule A (ACD).

Un mode de réalisation particulier de l'invention concerne les CSM-GW décongelées telle que décrites ci-dessus, pour leur utilisation dans le traitement du choc septique, la forme la plus grave du sepsis, qui est défini par l'apparition ou la persistance d'une hypotension artérielle et/ou de signes d'hypoperfusion périphérique malgré un remplissage vasculaire adéquat.

Dans un mode de réalisation avantageux, les CSM, de préférence les CSM-GW utilisées dans l'invention sont directement issues de la décongélation, sans remise en culture après la décongélation.

Contrairement aux études précédentes, dans lesquelles les cellules analysées sont des cellules fraîches, qui n'ont été soumises ni à une congélation, ni à une décongélation, etadministrées à des temps précoces chez les animaux dans un modèle de choc septique, les résultats de la présente invention apportent pour la première fois la preuve de l'efficacité des CSM-GW dans des conditions compatibles avec les conditions cliniques, à savoir l'administration des CSM-GW décongelées à un temps tardif dans l'évolution du sepsis.

La présente invention montre que les CSM-GW décongelées conservent les propriétés immunomodulatrices et antibactériennes à un niveau comparable avec celles des CSM isolées à partir de la moelle osseuse (CSM-MO) décongelées et que l'administration des GSM-GW permet même d'augmenter la survie des souris souffrant d'un choc septique par rapport à la survie des souris traitées par des CSM-MO.

Ces résultats montrent que les CSM-GW décongelées sont aptes à être utilisées immédiatement dans les conditions cliniques pour le traitement du choc septique.

Dans un mode de réalisation, les CSM, de préférence les CSM-GW utilisées dans l'invention sont lavées après décongélation. Dans un mode de réalisation, les CSM, de préférence les CSM-GW utilisées dans l'invention sont remises en suspension après décongélation. Dans un mode de réalisation, les CSM, de préférence les CSM-GW utilisées dans l'invention sont remises en suspension après décongélation dans une solution d'hydroxyéthylamidon. Dans un mode de réalisation, les CSM, de préférence les CSM-GW utilisées dans l'invention sont remises en suspension après décongélation dans une solution d'albumine. Dans un mode de réalisation, la solution d'albumine est une solution d'albumine 4%. Dans un mode de réalisation, la solution d'albumine comprend en outre du NaCl et/ou du citrate dextrose formule A (ACD).

Dans la présente invention, les Inventeurs constatent également pour la première fois une corrélation significative entre d'une part des facteurs obstétricaux et d'autre part la prolifération des CSM-GW et mettent à disposition les critères du choix des cordons ombilicaux pour obtenir des CSM-GW ayant une meilleure capacité de prolifération.

L'un des objets de l'invention concerne les CSM-GW issues d'un tissu de cordon ombilical humain répondant à ces critères.

Il est observé que ces critères améliorent la prolifération cellulaire en diminuant le temps de doublement du nombre de cellules, notamment au passage P1. Ces critères permettent également d'obtenir un plus grand nombre de cellules avec un temps de doublement court.

Dans un mode de réalisation avantageux, les CSM-GW utilisées dans la présente invention sont issues d'un tissu de cordon ombilical humain provenant d'une mère répondant à au moins l'un des critères suivants : ayant reçu une administration d'ocytocine lors de l'accouchement, ayant accouché par travail dirigé, ayant accouché à terme, n'ayant pas présenté de prééclampsie pendant la grossesse, dont l'enfant n'a pas présenté de troubles néonataux et ayant subi une imprégnation tabagique au cours de la grossesse.

Les Inventeurs ont constaté que ces facteurs obstétricaux présentent un impact positif sur la prolifération cellulaire des CSM, de préférence des CSM-GW et permettent de sélectionner des CSM, de préférence des CSM-GW avec de meilleures propriétés de prolifération.

Conformément à un mode de réalisation, les CSM, de préférence les CSM-GW utilisées dans la présente invention sont des cellules de grade clinique.

Par conséquent, tout milieu de culture et réactif utilisés dans la présente invention sont dépourvus de substance d'origine animale non humaine. Dans un mode de réalisation, tout milieu de culture et réactif utilisés sont dépourvus de sérum d'origine animale non humaine. Dans un mode de réalisation, tout milieu de culture et réactif utilisés sont dépourvus de sérum de quelque origine, même humain.

Avantageusement, le milieu de culture utilisé dans la présente invention pour l'adhésion de cellules contient du lysat plaquettaire humain.

La présente invention a également pour objet une composition comprenant des CSM décongelées, de préférence des CSM-GW décongelées telles que décrites ci-dessus, pour son utilisation dans le traitement du sepsis.

La présente invention a également pour objet une composition pharmaceutique comprenant à titre de principes actifs des cellules souches mésenchymateuses décongelées de grade clinique issues de la gelée de Wharton telles que décrites ci-dessus, pour son utilisation dans le traitement du sepsis.

Ladite composition comprend en outre un excipient pharmaceutiquement acceptable.

Conformément à la présente invention, un excipient pharmaceutiquement acceptable est un excipient dépourvu de substance d'origine animale non-humaine et adapté pour une utilisation en contact avec les cellules des individus humains sans toxicité, irritation, ou réponse allergique indue. L'homme du métier saura choisir un excipient pharmaceutiquement acceptable selon la formulation galénique de la composition et son mode d'administration.

À titre d'exemple, ledit excipient est de l'albumine 4% ou une solution d'hydroxyéthylamidon (HEA) 130/0,42.

Ladite composition pharmaceutique peut être sous forme d'un produit de perfusion et conditionnée dans une poche de perfusion.

La présente invention a également pour objet un médicament comprenant des CSM décongelées, de préférence des CSM-GW décongelées telles que décrites ci-dessus, pour son utilisation dans le traitement du sepsis.

Dans un mode de réalisation, les CSM, de préférence les CSM-GW utilisées dans l'invention, peuvent être administrées au patient par voie systémique ou locale.

Dans un mode de réalisation, les CSM, de préférence les CSM-GW utilisées dans l'invention, peuvent être administrées au patient par voie intraveineuse, intravasculaire, intracérébrale, parentérale, intrapéritonéale, épidurale, intra-spinale, intrastémale, intra-articulaire, intra-synoviale, intrathécale, intraartérielle, intracardiaque ou intramusculaire.

Dans un mode de réalisation, les CSM, de préférence les CSM-GW utilisées dans l'invention, peuvent être administrées au patient par injection en bolus (aussi dite injection rapide) ou par infusion continue (aussi dite injection lente).

Dans un mode de réalisation préférentiel, les CSM-GW décongelées de grade clinique pour leur utilisation dans le traitement du sepsis sont administrées par voie intraveineuse.

Conformément à la présente invention, les CSM-GW décongelées de grade clinique décrites auparavant peuvent être administrées à un patient souffrant du sepsis avec une dose d'environ de 0,3 ×10⁶ à 3×10⁶ cellules par kg de poids, notamment une dose de 1×10⁶ cellules par kg de poids. L'homme du métier peut ajuster la dose selon la gravité de l'infection, le poids du patient, le nombre ou la fréquence de l'administration.

Dans un mode de réalisation, les CSM, de préférence les CSM-GW utilisées dans l'invention, peuvent être administrées au patient à une dose allant de 10³ à 10⁹ CSM/kg, de préférence de 10⁴ à 10⁸ CSM/kg, de préférence de 10⁵ à 10⁷ CSM/kg.

Dans un mode de réalisation, les CSM, de préférence les CSM-GW utilisées dans l'invention, peuvent être administrées au patient à une dose de 1 × 10⁵ CSM/kg, 2 × 10⁵ CSM/kg, 3 × 10⁵ CSM/kg, 4 × 10⁵ CSM/kg, 5 × 10⁵ CSM/kg, 6 × 10⁵ CSM/kg, 7 × 10⁵ CSM/kg, 8 × 10⁵ CSM/kg, 9 × 10⁵ CSM/kg, 1 × 10⁶ CSM/kg, 2 × 10⁶ CSM/kg, 3 × 10⁶ CSM/kg, 4 × 10⁶ CSM/kg, 5 × 10⁶ CSM/kg, 6 × 10⁶ CSM/kg, 7 × 10⁶ CSM/kg, 8 × 10⁶ CSM/kg, 9 × 10⁶ CSM/kg, 1 × 10⁷ CSM/kg.

Dans un mode de réalisation, les CSM, de préférence les CSM-GW utilisées dans l'invention, peuvent être administrées au patient à une dose allant de 10³ à 10⁹ CSM/kg/jour, de préférence de 10⁴ à 10⁸ CSM/kg/jour, de préférence de 10⁵ à 10⁷ CSM/kg/jour.

Dans un mode de réalisation, les CSM, de préférence les CSM-GW utilisées dans l'invention, peuvent être administrées au patient à une dose de 1 × 10⁵ CSM/kg/jour, 2 × 10⁵ CSM/kg/jour, 3 × 10⁵ CSM/kg/jour, 4 × 10⁵ CSM/kg/jour, 5 × 10⁵ CSM/kg/jour, 6 × 10⁵ CSM/kg/jour, 7 × 10⁵ CSM/kg/jour, 8 × 10⁵ CSM/kg/jour, 9 × 10⁵ CSM/kg/jour, 1 × 10⁶ CSM/kg/jour, 2 × 10⁶ CSM/kg/jour, 3 × 10⁶ CSM/kg/jour, 4 × 10⁶ CSM/kg/jour, 5 × 10⁶ CSM/kg/jour, 6 × 10⁶ CSM/kg/jour, 7 × 10⁶ CSM/kg/jour, 8 × 10⁶ CSM/kg/jour, 9 × 10⁶ CSM/kg/jour, 1 × 10⁷ CSM/kg/jour.

Dans un mode de réalisation, les CSM, de préférence les CSM-GW utilisées dans l'invention, peuvent être administrées au patient à une dose allant de 10³ à 10⁹ CSM/jour, de préférence de 10⁴ à 10⁸ CSM/jour, de préférence de 10⁵ à 10⁷ CSM/jour.

Dans un mode de réalisation, les CSM, de préférence les CSM-GW utilisées dans l'invention, peuvent être administrées au patient à une dose de 1 × 10⁵ CSM/kg/jour, 2 × 10⁵ CSM/jour, 3 × 10⁵ CSM/jour, 4 × 10⁵ CSM/jour, 5 × 10⁵ CSM/jour, 6 × 10⁵ CSM/jour, 7 × 10⁵ CSM/jour, 8 × 10⁵ CSM/jour, 9 × 10⁵ CSM/jour, 1 × 10⁶ CSM/jour, 2 × 10⁶ CSM/jour, 3 × 10⁶ CSM/jour, 4 × 10⁶ CSM/jour, 5 × 10⁶ CSM/jour, 6 × 10⁶ CSM/jour, 7 × 10⁶ CSM/jour, 8 × 10⁶ CSM/jour, 9 × 10⁶ CSM/jour, 1 × 10⁷ CSM/jour.

Dans un mode de réalisation, les CSM, de préférence les CSM-GW utilisées dans l'invention, peuvent être administrées en une dose unique, ou en doses multiples espacées dans le temps.

Dans un mode de réalisation, les CSM, de préférence les CSM-GW utilisées dans l'invention, peuvent être administrées une fois par jour, deux fois par jour, trois fois par jour ou plus.

Dans un mode de réalisation, les CSM, de préférence les CSM-GW utilisées dans l'invention, peuvent être administrées tous les jours, tous les deux jours, tous les trois jours, tous les quatre jours, tous les cinq jours, tous les six jours.

Dans un mode de réalisation, les CSM, de préférence les CSM-GW utilisées dans l'invention, peuvent être administrées une fois par semaine, toutes les semaines, toutes les 2 semaines, toutes les 3 semaines.

Dans un mode de réalisation, les CSM, de préférence les CSM-GW utilisées dans l'invention, peuvent être administrées une fois par mois, tous les mois, tous les 2 mois, tous les 3 mois, tous les 4 mois, tous les 5 mois, tous les 6 mois ou plus.

Conformément à l'invention, les CSM-GW décongelées décrites auparavant peuvent être diluées avant utilisation dans un excipient convenable, par exemple une solution d'albumine 4% (4 g/100 mL) ou une solution d'hydroxyéthylamidon (HEA) 130/0,42.

Les CSM-GW décongelées pour le traitement du sepsis selon la présente invention peuvent être utilisées seules, ou avant, pendant, ou après un autre traitement du sepsis, notamment un traitement antibiotique.

Dans un mode de réalisation, les traitements du sepsis sont bien connus de l'homme du métier, et incluent, sans y être limités, l'antibiothérapie, l'antifongithérapie, le remplissage vasculaire, l'administration d'agents vasopresseurs, et la corticothérapie.

Dans un mode de réalisation avantageux, les CSM-GW décongelées de grade clinique pour leur utilisation dans le traitement du sepsis sont administrées chez les patients souffrant du sepsis sous traitement d'antibiotique.

Dans un mode de réalisation, le patient est sous traitement d'au moins un antibiotique. Dans un mode de réalisation, le patient est sous traitement d'au moins deux antibiotiques. Dans un mode de réalisation, le patient est sous traitement d'au moins 3 ou plus antibiotiques.

Des exemples d'antibiotiques pour le traitement du sepsis incluent, sans y être limités, l'ampicilline, l'azithromycine, l'aztréonam, la céfazoline, la céfépime, la clindamycine, la lévofloxacine, le linézolide, le méropénèm, le métronidazole, le pipéracilline, le tazobactam, la tobramycine et la vancomycine.

Dans un mode de réalisation, les CSM-GW décongelées de grade clinique pour leur utilisation dans le traitement du sepsis sont administrées chez les patients souffrant du sepsis sous traitement d'antifongiques.

Dans un mode de réalisation, le patient est sous traitement d'au moins un antifongique. Dans un mode de réalisation, le patient est sous traitement d'au moins deux antifongiques. Dans un mode de réalisation, le patient est sous traitement d'au moins 3 ou plus antifongiques.

Des exemples d'antifongiques pour le traitement du sepsis incluent, sans y être limités, l'amphotéricine B déoxycholate, l'anidulafungine, la caspofungine, le fluconazole, l'itraconazole, la micafungine et le voriconazole.

Dans un mode de réalisation, les CSM-GW décongelées de grade clinique pour leur utilisation dans le traitement du sepsis sont administrées chez les patients souffrant du sepsis sous remplissage vasculaire.

Le remplissage vasculaire permet de corriger l'hypovolémie, de maintenir la pression artérielle moyenne supérieur à 65 mmHg et de limiter les signes cliniques d'hypoperfusion, en restaurant le volume intravasculaire. Le remplissage vasculaire est effectué au moyen de solutés.

Des exemples de solutés pour le remplissage vasculaire dans le traitement du sepsis incluent, sans y être limités, les colloïdes et les cristalloïdes.

Dans un mode de réalisation, les CSM-GW décongelées de grade clinique pour leur utilisation dans le traitement du sepsis sont administrées chez les patients souffrant du sepsis sous traitement d'agents vasopresseurs.

Dans un mode de réalisation, le patient est sous traitement d'au moins un agent vasopresseur. Dans un mode de réalisation, le patient est sous traitement d'au moins deux agents vasopresseurs. Dans un mode de réalisation, le patient est sous traitement d'au moins 3 ou plus agents vasopresseurs.

Des exemples d'agents vasopresseurs pour le traitement du sepsis incluent, sans y être limités, les catécholamines (incluent, la noradrénaline et l'adrénaline) et la vasopressine.

Dans un mode de réalisation, les CSM-GW décongelées de grade clinique pour leur utilisation dans le traitement du sepsis sont administrées chez les patients souffrant du sepsis sous traitement de corticoïdes.

Dans un mode de réalisation, le patient est sous traitement d'au moins un corticoïde. Dans un mode de réalisation, le patient est sous traitement d'au moins deux corticoïdes. Dans un mode de réalisation, le patient est sous traitement d'au moins 3 ou plus corticoïdes.

Des exemples de corticoïdes pour le traitement du sepsis incluent, sans y être limités, la cortisone, l'hydrocortisone et la prednisone.

D'autres traitements sont adaptés au sepsis, et sont bien connus de l'homme du métier. Des exemples de tels traitements incluent, sans y être limités, l'AB103, l'alcaline phosphatase recombinante recAP, le CaCP29, la drotrécogine alfa activée, l'EA-230, l'eritoran, l'esmolol, le GM-CSF, l'IFNγ, le lenercept, le lévosimendan, le LR12, la sélépressine, la talactoferrine alpha et la thrombomoduline humaine recombinante.

Un autre aspect de l'invention concerne un procédé de préparation de cellules souches mésenchymateuses de grade clinique issues de la gelée de Wharton. Le procédé de l'invention vise à produire des CSM-GW ayant une meilleure capacité de prolifération.

Dans un mode de réalisation, le procédé de l'invention vise à produire des CSM-GW tels que définies dans la présente invention. Dans un mode de réalisation, le procédé de l'invention vise à produire des CSM-GW tels que définies dans la présente invention, pour leur utilisation dans le traitement du sepsis.

Ledit procédé comprend les étapes suivantes :
(i) cultiver un tissu de cordons ombilicaux humains contenant la gelée de Wharton pour l'adhésion de cellules ;
(ii) incuber les cellules adhérentes obtenues à l'étape (i) dans un milieu contenant du lysat plaquettaire.

Ledit procédé comprend les étapes suivantes :
(i) cultiver un tissu de cordons ombilicaux humains contenant la gelée de Wharton dans un milieu de culture pour l'adhésion de cellules ;
(ii) incuber les cellules adhérentes obtenues à l'étape (i) dans un milieu contenant du lysat plaquettaire.

Dans un mode de réalisation, ledit procédé comprend les étapes suivantes :
(i) cultiver un tissu de cordons ombilicaux humains contenant la gelée de Wharton dans un milieu de culture de grade clinique pour l'adhésion de cellules ;
(ii) incuber les cellules adhérentes obtenues à l'étape (i) dans un milieu contenant du lysat plaquettaire ;
caractérisé en ce que ledit tissu provient d'une mère répondant à au moins l'un des critères suivants : ayant reçu une administration d'ocytocine lors de l'accouchement, ayant accouché par travail dirigé, ayant accouché à terme, n'ayant pas présenté de prééclampsie pendant la grossesse, dont l'enfant n'a pas présenté de troubles néonataux et ayant subi une imprégnation tabagique au cours de la grossesse.

Le susdit critère de sélection des cordons ombilicaux relatifs à la mère permet d'obtenir des CSM-GW avec de meilleures propriétés de prolifération.

Conformément à l'invention, les tissus de cordons ombilicaux humains sont collectés aux maternités immédiatement après l'accouchement à partir d'une mère répondant aux critères ci-dessus.

Dans un mode de réalisation, les cordons ombilicaux humains sont sectionnés et cultivés. Dans un mode de réalisation, les artères et la veine ombilicales sont retirées avant culture des cordons ombilicaux. Dans un mode de réalisation, les artères et la veine ombilicales ne sont pas retirées avant culture des cordons ombilicaux.

Dans un mode de réalisation, les cordons ombilicaux sont lavés avant leur mise en culture. Dans un mode de réalisation, les cordons ombilicaux sont rincés dans une solution d'a-MEM, de préférence dans une solution d'a-MEM de grade clinique. Dans un mode de réalisation, la solution d'a-MEM, de préférence la solution d'a-MEM de grade clinique, comprend en outre au moins un antibiotique et/ou au moins un antifongique. Dans un mode de réalisation, ledit au moins un antibiotique est sélectionné parmi la gentamicine, l'amoxicilline et la vancomycine. Dans un mode de réalisation, ledit au moins un antifongique est l'amphotéricine B.

Dans un mode de réalisation, les cordons ombilicaux sont rincés avant leur mise en culture. Dans un mode de réalisation, les cordons ombilicaux sont rincés dans une solution de PBS.

Dans un mode de réalisation, la culture de tissus de cordons ombilicaux humains contenant la gelée de Wharton pour l'adhésion de cellules est une culture à sec. Dans un mode de réalisation, le milieu de culture, de préférence le milieu de culture de grade clinique pour l'adhésion de cellules utilisé à l'étape (i) est un milieu α-MEM.

Dans un mode de réalisation, le milieu contenant du lysat plaquettaire utilisé à l'étape (ii) est un milieu α-MEM. Dans un mode de réalisation, le milieu contenant du lysat plaquettaire utilisé à l'étape (ii) comprenant en outre de 1% à 20% de lysat plaquettaire, de préférence de 1% à 15%, de préférence de 1% à 10%, de préférence de 2.5% à 7.5%, de préférence environ 5% de lysat plaquettaire. Dans un mode de réalisation, le milieu contenant du lysat plaquettaire utilisé à l'étape (ii) comprenant en outre au moins un antibiotique. Dans un mode de réalisation, ledit au moins un antibiotique est la gentamycine. Dans un mode de réalisation, le milieu contenant du lysat plaquettaire utilisé à l'étape (ii) comprenant en outre de l'héparine.

Dans un mode de réalisation, l'étape (ii) du procédé de l'invention est réalisée en condition d'hypoxie, de préférence à environ 5% CO₂/5% O₂. Dans un mode de réalisation, l'étape (ii) du procédé de l'invention est réalisée à 37°C.

Dans un mode de réalisation, pendant la mise en œuvre de l'étape (ii) du procédé de l'invention, les cellules sont cultivées jusqu'à 80% +/-10% de confluence.

Avantageusement, l'étape (ii) dudit procédé est répétée plusieurs fois, notamment 2 ou 3 fois. Dans la présente invention, il sera dénommé « **passage** » chaque répétition de l'étape (ii) du procédé. Par exemple, la première mise en œuvre de l'étape (ii) du procédé correspond au passage P0 ; sa première répétition correspond au premier passage (ou passage P1) ; sa seconde répétition correspond au second passage (ou passage P2) ; etc.

Conformément à l'invention, lorsque l'étape (ii) est répétée, après obtention de 80% +/-10% de confluence, les cellules sont détachées par toute technique connue de l'homme du métier, notamment par action de la trypsine de grade clinique.

Conformément à l'invention, les cellules récoltées à la fin de l'étape (ii) sont cryoconservées à environ -20°C, de préférence à environ -80°C, de préférence entre - 150°C et -196°C, de préférence à environ -150°C.

### BRÈVE DESCRIPTION DES FIGURES

La présente invention est illustrée plus en détail dans les figures et les exemples décrits ci-après.
**Figures 1A et 1B** : ces figures représentent respectivement le nombre moyen de CFU en anaérobie (Figure 1A) ou en aérobie (Figure 1B) par milligramme de rate. Les résultats sont présentés sous forme de moyenne ± SEM. n=7-12 souris par groupe. ^{∗}p<0,05 CSM versus PBS.
**Figure 2A et 2B** : ces figures représentent respectivement le nombre de CFU en anaérobie (Figure 2A) ou en aérobie (Figure 2B) par ml de sang. Les résultats sont présentés sous forme de moyenne ± SEM. n=7-12 souris par groupe. ^{∗}p<0,05 CSM versus PBS.
**Figures 3A, 3B****,** **3C, 3D** **:** ces figures représentent respectivement la cinétique des neutrophiles dans le poumon (Figure 3A), la rate (Figure 3B), le foie (Figure 3C), le fémur (Figure 3D). Les résultats sont présentés sous forme de moyenne ± SEM. n=4-6 souris par groupe. ^{∗}p<0,05 CSM versus PBS ; ^{∗∗}p<0,01 CSM versus PBS ; ^{∗∗∗}p<0,001 CSM versus PBS.
**Figure 4A, 4B****,** **4C, 4D** **:** ces figures représentent respectivement la cinétique des monocytes dans le poumon (Figure 4A), le fémur (Figure 4B), ou des monocytes inflammatoires dans le poumon (Figure 4C), le fémur (Figure 4D). Les résultats sont présentés sous forme de moyenne ± SEM. n=4-6 souris par groupe. ^{∗}p<0,05 CSM versus PBS ; ^{∗∗}p<0,01 CSM versus PBS ; ##p<0,01 CSM-MO versus CSM-GW.
**Figure 5** : cette figure montre le taux de survie après ligature et perforation du *cæcum* des souris traitées par CSM-GW, CSM-MO, et des souris non-traitées. Les résultats sont présentés sous forme de courbes de Kaplan-Meier. n=18-25 souris par groupe. ^{∗}p<0,05 CSM versus PBS.

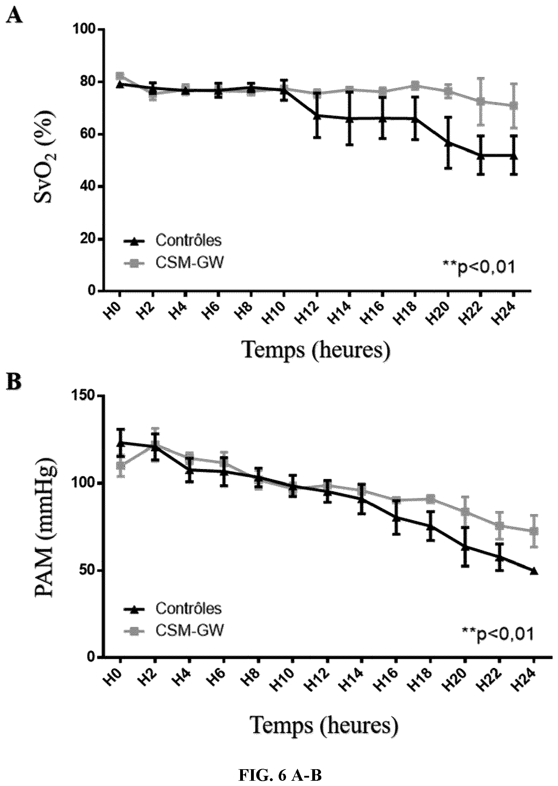

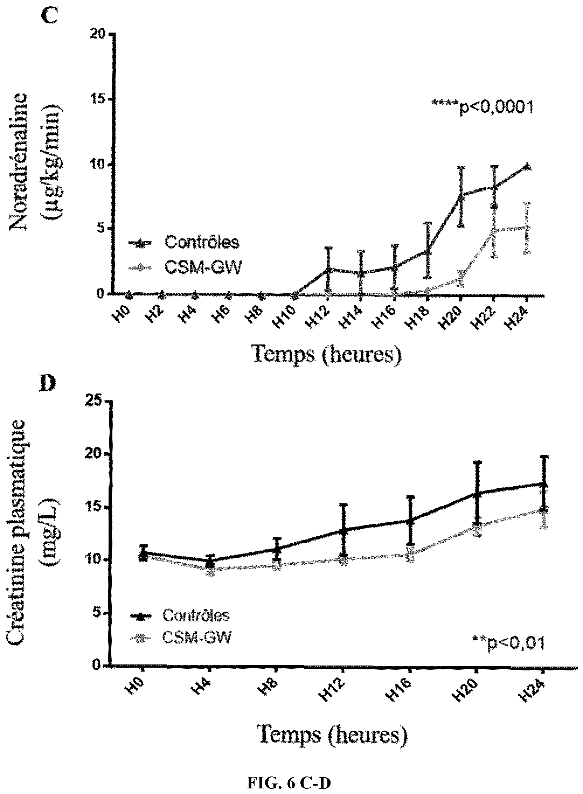

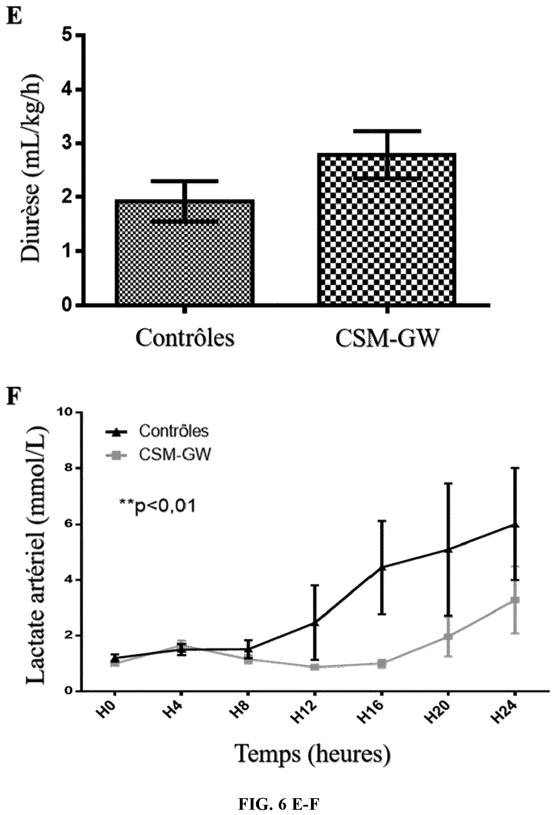

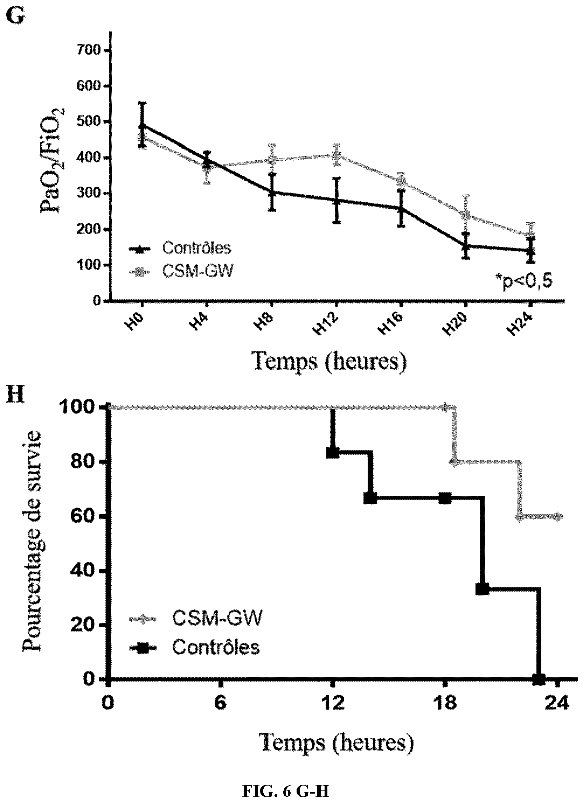

**Figures 6A, 6B****,** **6C, 6D****,** **6E, 6F****,** **6G, 6H** : ces figures montrent l'impact du traitement par CSM-GW sur le sepsis chez le cochon par rapport aux cochons sans traitement. La figure 6A compare la saturation veineuse en oxygène en fonction du temps dans le groupe avec traitement de CSM-GW et dans le groupe sans traitement (^{∗∗}p<0,01 CSM-GW *versus* contrôle). La figure 6B compare la diminution de pression artérielle moyenne en fonction du temps dans le groupe avec traitement de CSM-GW et dans le groupe sans traitement (^{∗∗}p<0,01 CSM-GW *versus* contrôle). La figure 6C compare le taux de noradrénaline administrée en fonction du temps dans le groupe avec traitement de CSM-GW et celle dans le groupe sans traitement (^{∗∗∗∗}p<0,0001 CSM-GW *versus* contrôle). La figure 6D compare le taux de créatinine plasmatique en fonction du temps dans le groupe avec traitement de CSM-GW et dans le groupe sans traitement (^{∗∗}p<0,01 CSM-GW *versus* contrôle). La figure 6E compare la diurèse dans le groupe avec traitement de CSM-GW et dans le groupe sans traitement. La figure 6F compare le taux de lactatémie en fonction du temps dans le groupe avec traitement de CSM-GW et dans le groupe sans traitement (^{∗∗}p<0,01 CSM-GW *versus* contrôle). La figure 6G compare le rapport PaO₂/FiO₂ en fonction du temps dans le groupe avec traitement de CSM-GW et dans le groupe sans traitement (^{∗}p<0,5 CSM-GW *versus* contrôle). La figure 6H compare la survie des animaux en fonction du temps dans le groupe avec traitement de CSM-GW et dans le groupe sans traitement.
**Figures 7A, 7B** : ces figures montrent l'impact de facteurs obstétricaux sur la prolifération cellulaire (Figures 7A et 7B). Le temps de doublement est inversement proportionnel à la prolifération. ^{∗}: p ≤ 0.05 en régression bivariée, ^{∗∗}: p ≤ 0.05 en régression multivariée.

### EXEMPLES

### Exemple 1 : Production des CSM-GW

### Isolement des CSM-GW

Les cordons ombilicaux humains sont collectés à la Maternité du CHRU de Nancy après avoir informé les mères donneuses et avoir obtenu leur consentement écrit. Les cordons sont placés dans un milieu de prélèvement et conservés à 4°C. Le milieu de prélèvement, placé dans une boite stérile (par exemple, Cryokits, Verreries Talançonnaises), est composé de tampon phosphate salin (PBS, Macopharma, BC0120020) afin d'assurer un pH proche de 7, d'héparine afin d'éviter que le sang résiduel ne coagule, d'antibiotique (Gentamicine). Le cordon peut être conservé 24h à 4°C dans le milieu de transport après prélèvement.

À réception, les cordons ombilicaux sont immergés une heure à température ambiante dans une solution d'antibiotiques-antifongique composée de gentamicine, amoxicilline, vancomycine et amphotéricine B dans du milieu de culture α-MEM de grade clinique (Macopharma, BC0110010) avec une concentration finale respective de 0,5g/L; 1g/L ; 1g/L et 0,05g/L. Ce bain permet de réduire les risques de développement microbien au cours de la culture qui serait secondaire à une contamination durant le prélèvement.

Une fois le cordon décontaminé, la veine ainsi que la partie externe du cordon sont lavées au PBS. 10 mL de PBS sont injectés à l'intérieur de la veine ombilicale à l'aide d'une seringue afin d'enlever le sang résiduel. Le cordon est ensuite prédécoupé en morceaux de 5 cm à l'aide d'un scalpel stérile puis de fines coupes transversales (2 à 3 mm d'épaisseur) sont réalisées. Ces morceaux sont ensuite placés dans des boîtes de culture à couvercle amovible (TPP, 90552). L'adhérence à sec des morceaux au fond des boites est réalisée pendant 15 minutes puis du milieu complet, composé d'a-MEM supplémenté avec 5% de lysat plaquettaire (Macopharma, BC0190020) en présence de gentamycine et d'héparine, est ajouté. La culture est réalisée en incubateur dédié à 37°C et 5% de CO₂ et en hypoxie (5% d'O₂). Après 5 jours, le milieu de culture est changé.

Après 10 jours de culture environ, les CSM-GW auront migré et adhéré au fond des boites de culture. Les morceaux sont alors retirés à l'aide d'une pince stérile et la boite est lavée avec du tampon PBS. Les cellules sont ensuite cultivées jusqu'à confluence (>80%) entre 15 jours et 3 semaines (entre J14 et J21).

### Culture des CSM-GW

Après obtention de la confluence, les cellules sont détachées par action de la trypsine recombinante GMP (TrypLE, Invitrogen).

Après lavage, les cellules sont réensemencées à 1×10³ cellules par cm² en passage 1 dans du milieu complet. Une unité de culture à 2 étages de 1270 cm² de surface de type CellSTACK (CELLSTACK2, Macopharma) est utilisée ainsi qu'un bouchon universel avec cheminée (Bouchon MPC, Macopharma). Le kit d'ensemencement (BC0400011, Macopharma) comporte le système de tubulures permettant l'apport des différents composants du milieu et des cellules, ainsi que la connectique pour le raccordement au contenant de culture.

Le milieu est changé 1 fois par semaine jusqu'à obtention de la confluence (> 80%) (entre J7 et J8 à partir de la mise en culture). Pour cela, un kit de changement de milieu (BC0400021, Macopharma) comportant le système de tubulures permettant l'apport des différents composants du milieu, une poche poubelle, ainsi que la connectique pour le raccordement au contenant de culture est utilisé, ainsi qu'un bouchon universel avec cheminée. Cependant, la plupart du temps, le passage P1 dure 1 semaine et le changement de milieu n'est pas nécessaire.

À l'obtention de la confluence, les cellules sont cryoconservées en une banque de cellules primaires (en anglais, *Master Cell Bank* ou *MCB*).La Food and Drug Administration (FDA) définit une MCB comme une collection de cellules de composition uniforme, dérivée d'un tissu ou d'une cellule unique, et stockée en aliquot sous des conditions définies.

### Recueil final des cellules

Après l'obtention de la confluence à la fin du passage P1, les cellules sont recueillies par action de la trypsine comme décrit précédemment puis lavées. Un contrôle de viabilité et de numération cellulaire ainsi que les différents contrôles de P1 sont réalisés (Cf tableau ci-après). Les cellules sont ensuite congelées.

### Conservation des CSM de la MCB

Les CSM sont stockées dans des cuves (en vapeur d'azote) à -150°C avec enregistrement continu de la température et du niveau d'azote. De plus, 5 à 10 tubes échantillons de CSM sont également congelés pour la réalisation des contrôles.

### Décongélation de la MCB et lavage des CSM

Les CSM sont décongelées et lavées en technique de type Sepax (Biosafe) puis réensemencées en passage 2 puis 3 selon le même protocole que pour le P1.

### Recueil final des cellules

Après l'obtention de la confluence à la fin du passage P3, les cellules sont recueillies par action de la trypsine comme décrit précédemment puis lavées. Un contrôle de viabilité et de numération cellulaire ainsi que les différents contrôles de fin de production sont réalisés (Cf tableau ci-après). Les cellules sont ensuite congelées en banque de cellules de travail (en anglais, *Working Cell Bank* ou *WCB*)*.* La Food and Drug Administration (FDA) définit une WCB comme une collection de cellules dérivées d'un ou plusieurs aliquot d'une MCB. Les cellules de la MCB sont expansées par sous-cultures en série jusqu'au passage sélectionné, après quoi les cellules sont combinées, concentrées, et aliquotées. Un ou plusieurs aliquots de la WCB ainsi obtenus peuvent être utilisés pour produire un lot du produit final en vue de son utilisation.

### Conservation des CSM de la WCB

Les CSM sont stockées dans des cuves (en vapeur d'azote) à -150°C avec enregistrement continu de la température et du niveau d'azote. De plus, 5 à 10 tubes échantillons de CSM sont également congelés pour la réalisation des contrôles.

### Décongélation, lavage des CSM de la WCB et acheminement des CSM

Les CSM sont décongelées et lavées en technique de type Sepax (Biosafe) puis reprises dans un volume de 75 mL avec de l'Albumine 4% et conservées entre 4 et 10°C. Les CSM sont conditionnées en poche (poche de 150 ml), étiquetée selon le mode opératoire réglementaire ; ladite poche est placée dans un emballage secondaire (poche plastique) lui-même également étiqueté puis dans un container de transport avec un enregistreur de température, un certificat de validation, une notice d'injection et une fiche de tolérance après administration. Le transport des CSM vers le site d'administration est effectué par un transporteur agréé par le CHRU.

### Contrôles qualité effectués au décours du processus de production

Afin de garantir la stérilité et la sécurité du produit à administrer, des contrôles du produit sont effectués à chaque étape du processus de culture. L'ensemble des contrôles qualité sont répertoriés dans le **Tableau 1.**

**Tableau 1**

| **Etapes** | **Contrôles effectués à chaque étape** |
|---|---|
| **Réception du prélèvement** : | Marqueurs infectieux de la donneuse (Sérologies / DGV) |
| **Changement de milieu** Le milieu est changé 1 fois / semaine | Contrôle de stérilité |
| **Trypsination : fin P0** | Numération |
| | Viabilité cellulaire |
| | Phénotype cellulaire |
| | Contrôle de stérilité |
| | CFU-F |
| **Trypsination : fin P1 MCB** | Numération |
| | Viabilité cellulaire |
| | Phénotype cellulaire |
| | Contrôle de stérilité |
| | Contrôle de l'absence d'endotoxine et de mycoplasmes |
| | CFU-F |
| | Contrôle de la différenciation en cellules des tissus mésodermiques |
| | Télomérase (hTERT) |
| | Caryotype |
| | Culture mixte lymphocytaire |
| | Test de Potency (en cours de définition) |
| **Trypsination** : **fin P2** | Numération |
| | Viabilité cellulaire |
| | Phénotype cellulaire |
| | Contrôle de stérilité |
| | CFU-F |
| **Recueil final : fin P3 (pré-congélation)** Après l'obtention de la confluence à la fin de P₃, les CSM sont trypsinées, lavées puis congelées. | Numération |
| | Viabilité cellulaire |
| | Phénotype cellulaire |
| | Contrôle de stérilité |
| | Contrôle de l'absence d'endotoxine et de mycoplasmes |
| | CFU-F |
| | Contrôle de la différenciation en cellules des tissus mésodermiques |
| | Télomérase (hTERT) |
| | Caryotype |
| | Culture mixte lymphocytaire |
| | Test de Potency (en cours de définition) |

### Contrôles cellulaires

### Numération cellulaire

Sur le produit de départ et à chaque étape de recueil de cellules (P0 et P3), la numération cellulaire est déterminée à l'aide d'un hématimètre après lyse des hématies.

### Viabilité cellulaire

Sur le produit de départ et à chaque étape de recueil de cellules, la viabilité est déterminée par cytométrie en flux par marquage au 7 AAD en fin de P0, P1, P2, P3 doit être >80 %.

### Phénotype cellulaire

L'expression des marqueurs de surface caractéristiques d'un type cellulaire sera déterminée par cytométrie en flux après marquage des cellules avec des anticorps monoclonaux ou les contrôles isotypiques correspondants.

Au minimum 60 % des cellules doivent exprimer les antigènes suivants : CD90, CD73, CD105, CD44 et 80 % des cellules ne pas exprimer : CD34, CD11b, CD19, CD45, HLA-DR (et/ou IMF < 2 fois IMF du contrôle isotypique). Le marqueur CD106 doit être identifié au minimum sur 10% des cellules.

### CFU-F

La capacité clonogénique sera mesurée par la culture des progéniteurs fibroblastiques (colony forming unit fibroblast : CFU-F). Les CFU-F (> 50 cellules) seront numérées au microscope inversé au grossissement ×10, après fixation au méthanol et coloration par le Giemsa. Brièvement, 2.5 et 5 × 10² cellules sont ensemencées dans 5 mL de milieu dans un flacon de 25 cm². Le milieu est renouvelé dans sa totalité 2 fois par semaine. La culture est arrêtée à J10, elle est fixée et colorée au Giemsa. Les colonies de plus de 50 cellules sont alors comptées. La culture de CFU-F est réalisée à la fin de chaque passage.

### Contrôle immunologique

L'absence de capacité immunostimulante des CSM est contrôlée. Pour cela, une culture mixte lymphocytaire sera réalisée en utilisant les CSM produites et irradiées comme cellules stimulantes et les cellules mononucléées de 2 témoins sains. L'effet immunostimulant est évalué par la mesure de l'index de stimulation. Ces tests sont réalisés dans le secteur contrôle qualité du département médicaments de thérapie innovante préparés ponctuellement (MTI-PP) de l'Unité de Thérapie Cellulaire et banque de Tissus (UTCT) de Nancy. L'effet d'immunomodulation est évalué en ajoutant à une culture mixte lymphocytaire classique des CSM comme troisième partenaire.

### Contrôle microbiologique

### Contrôles des marqueurs infectieux du donneur

Un diagnostic génomique viral est effectué conjointement chez la donneuse pour le VIH, et le VHB et le VHC. Des marqueurs infectieux conformes sont : VIH : Test Combiné Ag P24+ Ac anti VIH 1+2 : Négatif, PCR VIH : Négatif, HBV : Ag HBs négatif, Ac anti HBc négatif, PCR HBV : Négatif, HCV : Ac anti HCV : Négatif, PCR HCV : Négatif, HTLV : Ac anti HTLV I+II : Négatif, Syphilis : Ac anti-TP : Négatif, CMV, EBV et Toxoplasmose : IgM négatives, IgG négatives ou positives.

### Bactériologie

Le contrôle microbiologique est effectué selon les recommandations de l'Agence nationale de sécurité du médicament et des produits de santé (ANSM) pour les Produits de Thérapie Cellulaire (PTC) à partir d'hémocultures aérobie et anaérobie réalisées en technique Bactec. Ce contrôle est réalisé à chaque étape de culture. Sur les CSM décongelées, les résultats des contrôles obtenus *a posteriori* de l'injection des CSM au patient doivent être négatifs. En cas de contrôle microbiologique positif après réinjection, une information du clinicien sera réalisée immédiatement, l'identification du germe sera communiquée ainsi que les résultats de l'antibiogramme conformément à la procédure interne au département MTI-PP.

### Autres contrôles

### L'absence de transcrit hTERT

L'absence d'activité Télomérase est recherché par qRT-PCR dans les CSM après culture (fin de P1 et P3). Ce contrôle sera réalisé sur un aliquot cryoconservé de 10⁶ CSM.

### Caryotype

Il est effectué sur un échantillon cellulaire frais de CSM obtenu en fin de culture (fin de P1 et P3), avant cryopréservation.

### Dosage de endotoxines et mycoplasmes

Il est effectué sur un échantillon cellulaire frais ou congelé de CSM obtenu en fin de culture (fin de P1 et P3), avant cryopréservation.

### Administration des CSM

Les cellules ainsi préparées sont administrées aux patients hospitalisés en réanimation présentant un choc septique ou un sepsis à une dose d'au moins 1×10⁶/kg CSMs hétérologues dans 75mL d'albumine 4%, NaCl, ACD, perfusés en 30 minutes sur voie veineuse centrale. Le traitement est reçu et administré sur voie veineuse centrale de préférence dans les 10 heures suivant l'étape de décongélation et lavage des CSM de la WCB, et au maximum jusqu'à 24 heures suivant l'étape de décongélation et lavage des CSM de la WCB.

### Modèle murin de choc septique

Un choc septique a été induit chez des souris C57BL/6 immunocompétentes par ligature et perforation du *cæcum* (CLP). Ce modèle, considéré comme le meilleur standard du choc septique murin, permet de mimer une péritonite humaine.

Après chirurgie, les souris ont été randomisées en 3 groupes : un groupe recevant 0,25×10⁶ CSM de GW humaines, un groupe recevant 0,25×10⁶ CSM de MO obtenues par culture à partir d'un prélèvement de Moelle osseuse d'un donneur sain ayant donné son consentement et un groupe témoin recevant du PBS.L'administration des CSM ainsi que du PBS a été réalisée 24 heures après le début du choc septique par voie intraveineuse au niveau du sinus rétro-orbital.

Les CSM-GW et CSM-MO sont utilisées juste après décongélation sans remise en culture préalable.

### Exemple 2 : Effet des CSM-GW sur le choc septique chez les souris

### Impact des CSM-GW sur la bactériémie

### Protocole

Les CSM-GW humaines décongelées sont utilisées.

Quarante-huit heures après le début du choc septique, soit 24h après l'administration de CSM, les souris ont été mises à mort par injection létale d'anesthésique. La rate et le sang des souris ont été prélevés puis ensemencés afin de dénombrer le nombre de CFU (colony forming unit).

La comparaison des différents groupes a été effectuée par un test de Kruskal-Wallis.

### Résultats

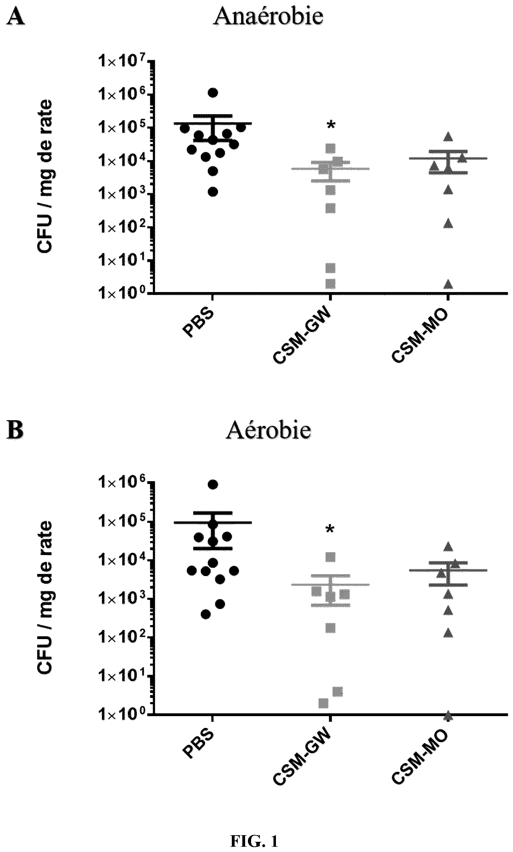

Deux jours après le choc septique seules les souris traitées par des CSM-GW ont présenté une diminution significative de la bactériémie et du nombre de CFU spléniques. Le nombre moyen de CFU par milligramme de rate ou de sang a été compté. Quarante-huit heures après la procédure CLP, le groupe traité par les CSM-GW a présenté en moyenne 2,3×10³ CFU en aérobie par mg de rate et 5,8.10³ CFU en anaérobie par mg de rate tandis que le groupe témoin et le groupe traité par CSM de MO ont présenté un nombre moyen de CFU en aérobie par mg de rate respectivement de 9,5×10⁴ et 5,5×10³ et un nombre moyen de CFU en anaérobie par mg de rate respectivement de 1,3×10⁵ et 1,1×10⁴ **(****Figures 1A et 1B****).**

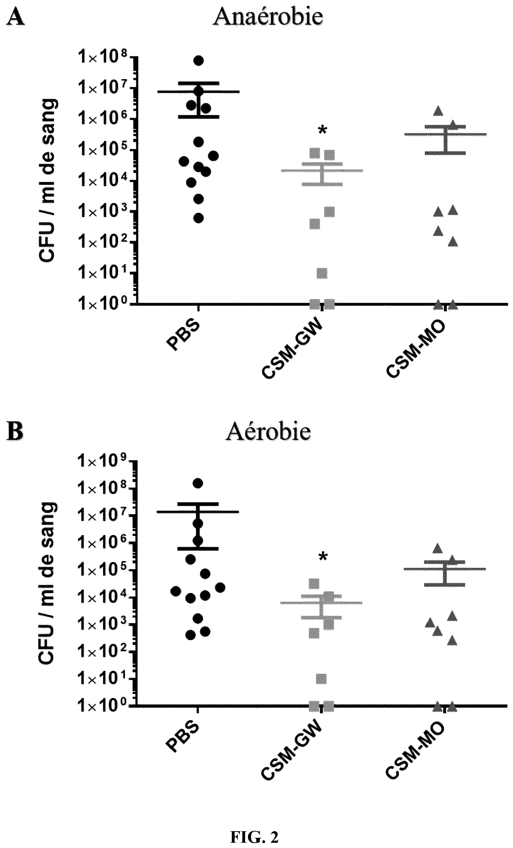

Les résultats obtenus dans le sang sont similaires **(****Figure 2A et 2B****).** Le groupe traité par les CSM issues de la gelée de Wharton a présenté en moyenne 6×10³ CFU en aérobie/mL de sang et 2,1×10⁴ CFU en anaérobies/mL de sang tandis que le groupe témoin et le groupe traité par CSM de MO ont présenté un nombre moyen de CFU en aérobie par mL de sang respectivement de 1,3×10⁷ et 1,1×10⁵ et un nombre moyen de CFU en anaérobie par mL de sang respectivement de 7,7×10⁶ et 3,2×10⁵.

### Conclusions

Ces résultats montrent pour la première fois une action antibactérienne des CSM issues de la gelée de Wharton dans le choc septique lorsqu'elles ont été utilisées juste après décongélation.

### Impact des CSM-GW sur l'afflux cellulaire au sein des organes

### Protocole

Quarante-huit heures ou 7 jours après l'induction du choc septique, les souris ont été mises à mort par injection intra-péritonéale de pentobarbital et les organes ont été prélevés. La rate et le foie ont été broyés puis filtrés. La moelle osseuse a été extraite du fémur en injectant rapidement 1 ml de PBS dans la cavité médullaire. Les poumons ont été coupés en fins morceaux puis placés dans 2 ml de collagénase pendant 45 minutes avant d'être broyés et filtrés. Les cellules extraites des différents organes ont été lavées par centrifugation. Une numération cellulaire a été effectuée.

Les cellules ont été marquées avec 5µL des anticorps anti CD45, CD11b, Ly6C, Ly6G afin d'identifier et de quantifier au sein des différents organes les monocytes totaux, les monocytes inflammatoires et anti-inflammatoires ainsi que les neutrophiles.

Le même protocole a été réalisé sur des souris saines avant l'induction du choc septique.

La comparaison des différents groupes a été réalisée par un test 2way ANOVA suivi d'un test Tukey. Une significativité statistique a été acceptée pour p<0,05.

### Résultats

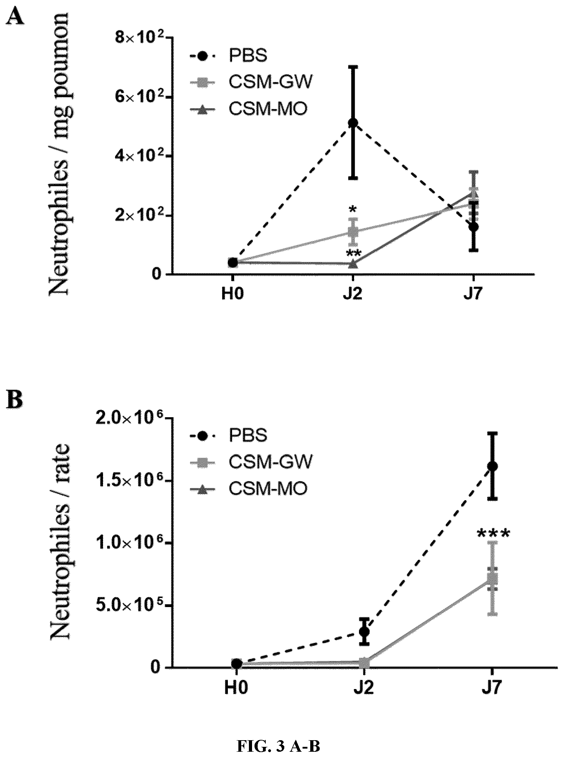

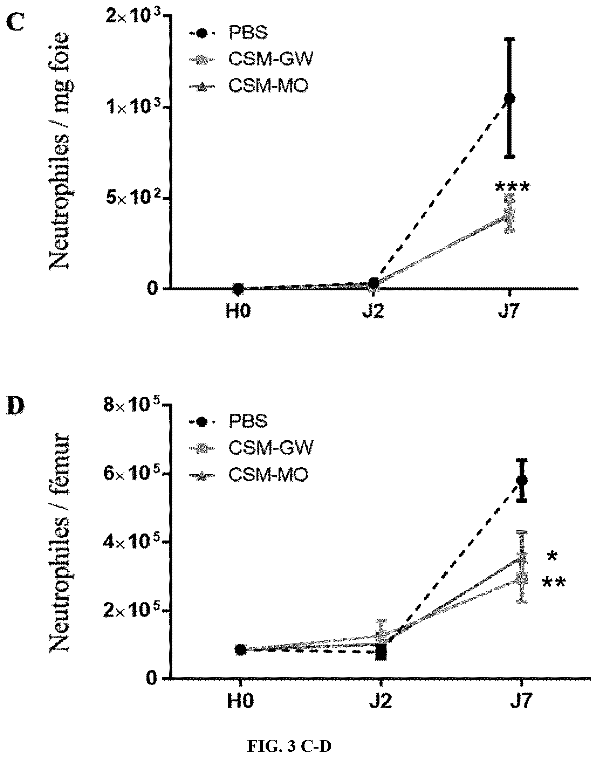

Deux jours après l'induction du choc septique, les souris témoins ayant reçu par voie intraveineuse une solution saline ont présenté un nombre de neutrophiles pulmonaires significativement plus important que les souris traitées par injection de cellules souches mésenchymateuses **(****Figure 3A****).** A J7 une accumulation significativement moins importante de neutrophiles a été notée au niveau des rates et foies des souris traitées par CSM de MO et de GW (p<0,001) **(****Figures 3B**, 3C). Sept jours après CLP le nombre de neutrophiles contenus dans les fémurs des animaux traités était significativement moins important comparativement au groupe témoin (PBS vs MO p<0,05 ; PBS vs GW p<0,01) **(****Figure 3D****).**

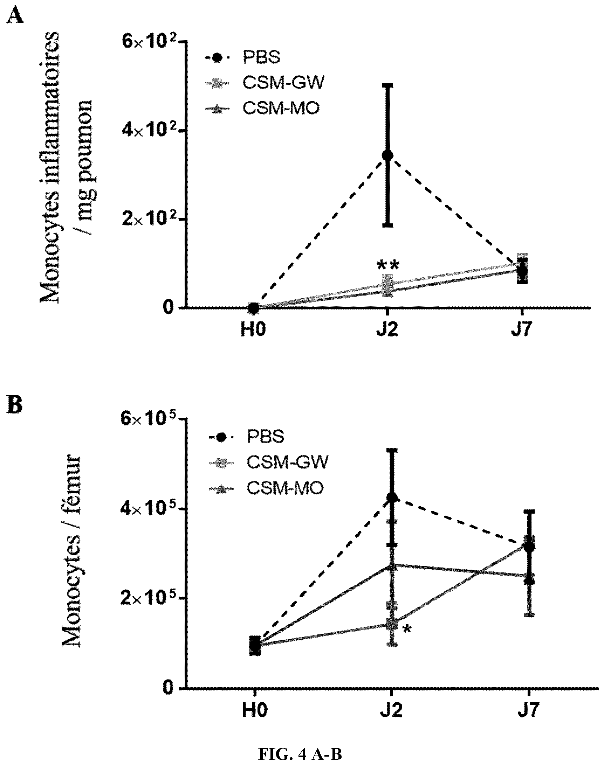

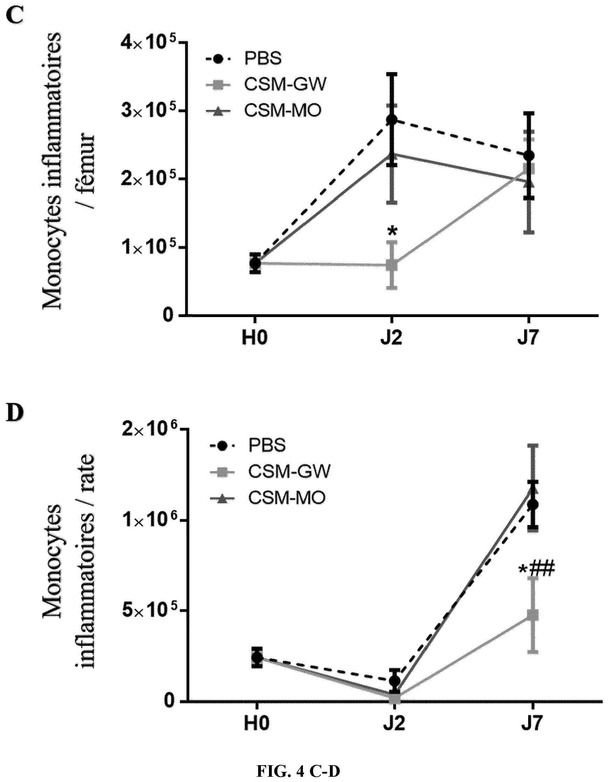

Deux jours après l'induction du choc septique et 24h après injection des CSM, les poumons des souris ayant reçu des CSM de MO ou de GW contenaient un nombre de monocytes inflammatoires ly6C^{high} significativement moins important que chez les souris témoins (p<0,01) **(****Figure 4A****).** Une diminution significative du nombre de monocytes totaux **(****Figure 4B****)** et du nombre de monocytes inflammatoires ly6C^{high} **(****Figure 4C****)** à J2 a également été retrouvée au niveau des fémurs des souris traitées par des CSM-GW (p<0,05) comparativement aux souris témoins tandis qu'aucune différence significative n'a été notée entre le groupe témoin et le groupe CSM-MO.

Sept jours après l'induction du choc septique un nombre de monocytes inflammatoires ly6C^{high} significativement moins important a été observé dans les rates des souris traitées par CSM-GW comparativement aux souris traitées par CSM-MO (p<0,01) et par PBS (<0,05) **(****Figure 4D****).**

Cette étude met en évidence que les CSM sont capables de moduler l'infiltrat leucocytaire dans un modèle de sepsis polymicrobien. Il est montré que l'action des CSM-GW décongelées ne se limite pas aux 48 premières heures suivant leur injection mais qu'elles sont capables d'avoir des effets bien plus tardifs sur le recrutement cellulaire au cours du choc septique.

En plus d'être efficaces à un temps tardif, les CSM présentent également une action précoce sur le trafic leucocytaire. Il est montré dans les résultats analysés ci-dessus que des CSM diminuent 48 h après l'induction du choc septique l'afflux de neutrophiles dans les poumons.

Ces résultats montrent que les CSM-GW, comme les CSM-MO, sont capables de diminuer l'accumulation au sein des organes de neutrophiles, impliqués dans le développement des défaillances d'organe associées au choc septique. En effet, il est connu que l'accumulation anormale de neutrophiles peut induire d'une part, une occlusion vasculaire conduisant à une hypoxémie et une hypoperfusion tissulaire et d'autre part engendrer des dysfonctions de la microcirculation par libération massive d'espèces réactives de l'oxygène.

Par ailleurs, contrairement aux CSM de MO, les CSM-GW sont en mesure de diminuer d'une part la production fémorale des monocytes totaux deux jours après le choc septique et d'autre part la production des monocytes pro-inflammatoires conduisant à une moindre accumulation de cette sous-population monocytaire dans la rate des souris. Etant donné que le choc septique modifie les propriétés des monocytes en induisant notamment une augmentation de leur production d'espèces réactives de l'oxygène concourant à augmenter le score SOFA (sepsis related organ failure assessment) (Martins *et al.,* 2008), la diminution de l'accumulation et de la production des monocytes totaux et pro-inflammatoires induite par l'injection de CSM-GW peut apporter un réel bénéfice dans le traitement du choc septique.

### Impact des CSM-GW sur la survie

### Protocole

Après induction du choc septique chez des souris C57Bl/6 par technique CLP, 150µl de NaCl ont été administrés en sous cutanée afin de permettre le remplissage vasculaire après chirurgie. Dans le but de se rapprocher au plus près de la clinique, l'ensemble des souris a reçu une dose de 50µg/g de poids corporel d'imipenème toutes les 12h en intrapéritonéal (Alcayaga-Miranda *et al.,* 2015).

Un test de Wilcoxon a été réalisé. Une différence significative a été admise pour p<0.05.

### Résultats

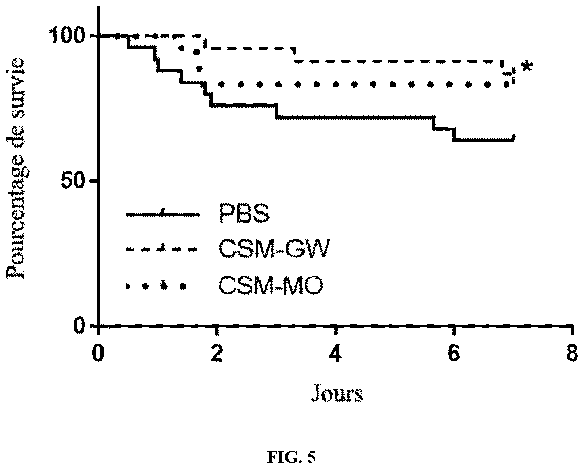

Le taux de survie est augmenté chez les souris traitées par CSM comparativement aux souris témoins. Un taux de survie de 64% est retrouvé chez les animaux contrôle tandis que 83% des animaux traités par des CSM-MO et 87% des animaux traités par des CSM-GW, ont survécu **(****Figure 5****).**

Les CSM-GW présentent un meilleur résultat en termes de survie dans un choc septique comparées aux CSM-MO.

### Exemple 3 : Etude de l'impact des CSM-GW sur le sepsis

L'efficacité des CSM-GW de grade clinique décongelées produites selon la méthode décrite dans la partie 1.1-1.6 ci-dessus est analysée chez le cochon, qui est l'animal le plus proche de l'homme d'un point de vue cardiovasculaire.

Quatre heures après l'induction d'une péritonite chez le cochon, une dose de CSM-GW de 1×10⁶/kg a été injectée par voie intraveineuse. Les CSM-GW ont été produites en grade clinique et ont été utilisées juste après décongélation. L'étude, menée durant les 24h suivant l'induction de la péritonite, a été effectuée en double aveugle et en présence d'un médecin réanimateur expérimenté. En conséquence, la prise en charge a été identique à celle d'un patient avec un maintien d'une part de la volémie et de la pression artérielle moyenne (>85mmHg) par remplissage vasculaire et par noradrénaline (maximum 10µg/kg/min) et d'autre part d'un débit cardiaque adéquat (>2l/min/m²) par dobutamine (maximum 20µg/kg/min).

Cette étude montre que l'administration de CSM-GW améliore significativement la saturation veineuse en oxygène témoignant d'une meilleure adéquation entre les apports en O₂ et la consommation en O₂ chez les animaux traités **(****Figure 6A****).**

L'administration de CSM-GW améliore les fonctions cardiovasculaires comme en témoignent l'amélioration de la pression artérielle moyenne (PAM) chez les animaux traités et l'administration plus tardive de noradrénaline **(****Figures 6B**, 6C). L'administration de CSM-GW améliore la fonction rénale : les animaux traités ont présenté une moindre augmentation de créatinine et une diurèse plus importante **(****Figures 6D**, 6E).

Par ailleurs, l'injection intraveineuse de CSM-GW diminue significativement la lactatémie dont la production supérieure à 2 mmol/l témoigne de l'hypoxie tissulaire **(****Figure 6F****).**

L'injection intraveineuse de CSM-GW augmente aussi le rapport PaO₂/FiO₂, qui reflète l'intensité du syndrome de détresse respiratoire aiguë **(****Figure 6G****).** Son augmentation chez les animaux traités met en évidence une moindre défaillance pulmonaire par rapport aux animaux non traités.

Il est observé dans cette expérience que l'augmentation de la survie des animaux traités par rapport aux animaux non-traités est d'environ 60% **(****Figure 6H****).**

### Exemple 4 : Analyse sur des facteurs obstétricaux

50 cordons ombilicaux ont été analysés en associant différents paramètres obstétricaux. Les CSM-GW sont isolées à partir de ces tissus selon la méthode décrite dans la partie 1.1-6 ci-dessus.

Après l'extraction des données, les 27 facteurs obstétricaux (14 relatifs à la mère, 6 au nouveau-né et 7 à l'accouchement) sont analysés pour les corrélations entre ces paramètres obstétricaux et 8 indicateurs biologiques de prolifération cellulaire (doublement de population). Chaque variable a fait l'objet d'une analyse de régression linéaire bivariée (RLB). Seuls les facteurs présentant une association significative au seuil 0,15 en RLB ont été candidats à une analyse de régression linéaire multivariée (RLM). La méthode de sélection des variables Stepwise a été utilisée avec un seuil d'entrée dans le modèle à 0,1 et un seuil de sortie du modèle à 0,05.

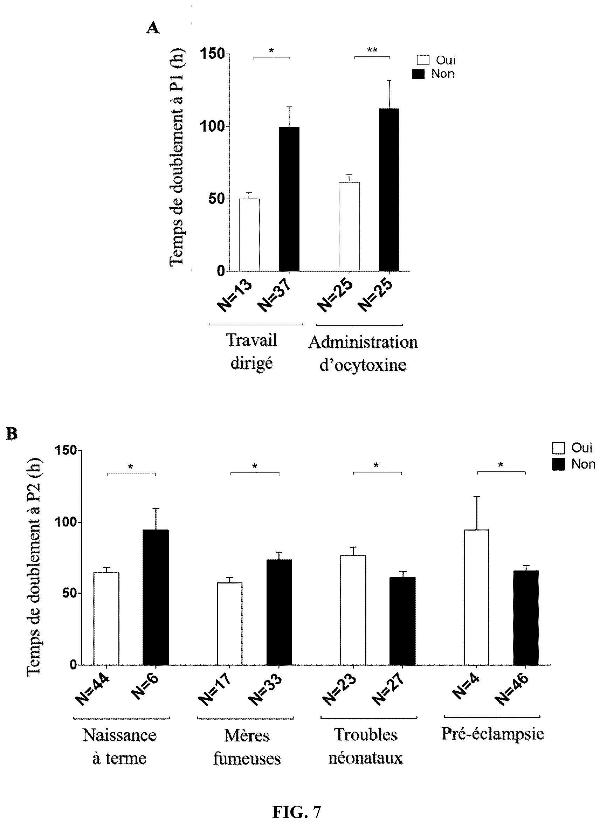

Après une analyse de régression linéaire multivariée, l'administration d'ocytocine au moment de l'accouchement a montré un impact positif sur la prolifération des CSM-GW en diminuant le temps de doublement (61,6 ± 5,2h vs 112,0 ± 19,5h, p = 0,0159) lors du premier passage P1 **(****Figure 7A****).** Cette administration d'ocytocine a également permis d'obtenir un plus grand nombre de cellules avec un temps de doublement court (<100 heures) (57,9% vs 25%, p = 0,0469). En ne considérant que les échantillons avec un temps de doublement en P1 inférieur à 100 heures, plusieurs facteurs ont présenté un impact positif sur le temps de doublement : travail dirigé (34,2% vs 0%, p = 0,0185), le nombre de semaines d'aménorrhée à la naissance (39,85 vs 37,92, p = 0,0212), tabagisme maternel (42,1% vs 8,3%, p = 0.0313) et le poids du placenta (552,24 vs 481,92, p = 0,0446). Au deuxième passage (P2), le poids à la naissance, le nombre de semaines d'aménorrhée, le poids du placenta, une grossesse normale et une absence de prééclampsie ont montré un impact positif sur la prolifération cellulaire **(****Figure 7B****).** Tous ces facteurs sont liés à la notion de naissance à terme et permettent de démontrer que des CSM-GW, provenant de nouveau-nés en bonne santé et nés à terme, présenteront des capacités prolifératives supérieures. Il est également observé que des CSM-GW issues de cordons ombilicaux de mères fumeuses présentent des capacités prolifératives augmentées.

L'identification de ces facteurs favorisant la prolifération des CSM-GW permet d'aider à la sélection de cordons ombilicaux qui renferment des CSM-GW avec de meilleures propriétés de prolifération.

### BIBLIOGRAPHIE

Alcayaga-Miranda, F., Cuenca, J., Martin, A., Contreras, L., Figueroa, F.E., and Khoury, M. (2015). Combination therapy of menstrual derived mesenchymal stem cells and antibiotics ameliorates survival in sepsis. Stem Cell Res. Ther. 6.
Chao, Y.-H., Wu, H.-P., Wu, K.-H., Tsai, Y.-G., Peng, C.-T., Lin, K.-C., Chao, W.-R., Lee, M.-S., and Fu, Y.-C. (2014a). An Increase in CD3+CD4+CD25+ Regulatory T Cells after Administration of Umbilical Cord-Derived Mesenchymal Stem Cells during Sepsis. PLoS ONE 9, e110338.
Chinnadurai, R., Copland, I. B., Garcia, M. A., Petersen, C. T., Lewis, C. N., Waller, E. K., Kirk, A. D., and Galipeau, J. (2016). Cryopreserved Mesenchymal Stromal Cells Are Susceptible to T-Cell Mediated Apoptosis Which Is Partly Rescued by IPNγ Licensing. Stem Cells, 34(9), 2429-2442.
Condor, J.M., Rodrigues, C.E., Sousa Moreira, R. d., Canale, D., Volpini, R.A., Shimizu, M.H.M., Camara, N.O.S., Noronha, I. d. L., and Andrade, L. (2016). Treatment With Human Whartons Jelly-Derived Mesenchymal Stem Cells Attenuates Sepsis-Induced Kidney Injury, Liver Injury, and Endothelial Dysfunction. Stem Cells Transl. Med. 5, 1048-1057.
Devaney, J., Horie, S., Masterson, C., Elliman, S., Barry, F., O'Brien, T., Curley, G.F., O'Toole, D., and Laffey, J.G. (2015). Human mesenchymal stromal cells decrease the severity of acute lung injury induced by E. coli in the rat. Thorax 70, 625-635.
Dombrovskiy, V.Y., Martin, A.A., Sunderram, J., and Paz, H.L. (2007). Rapid increase in hospitalization and mortality rates for severe sepsis in the United States: A trend analysis from 1993 to 2003∗: Crit. Care Med. 35, 1244-1250.
François M, Copland IB, Yuan S, Romieu-Mourez R, Waller EK, Galipeau J (2012). Cryopreserved mesenchymal stromal cells display impaired immunosuppressive properties as a result of heat-shock response and impaired interferon-γ licensing. Cytotherapy. 14(2): 147-52.
Gonzalez-Rey, E., Anderson, P., Gonzalez, M.A., Rico, L., Büscher, D., and Delgado, M. (2009). Human adult stem cells derived from adipose tissue protect against experimental colitis and sepsis. Gut 58, 929-939.
Iskander, K.N., Osuchowski, M.F., Stearns-Kurosawa, D.J., Kurosawa, S., Stepien, D., Valentine, C., and Remick, D.G. (2013). Sepsis: Multiple Abnormalities, Heterogeneous Responses, and Evolving Understanding. Physiol. Rev. 93, 1247-1288.
Kim, H., Darwish, I., Monroy, M.-F., Prockop, D.J., Liles, W.C., and Kain, K.C. (2014). Mesenchymal stromal (stem) cells suppress pro-inflammatory cytokine production but fail to improve survival in experimental staphylococcal toxic shock syndrome. BMC Immunol. 15, 1.
Krasnodembskaya, A., Song, Y., Fang, X., Gupta, N., Serikov, V., Lee, J.-W., and Matthay, M.A. (2010). Antibacterial Effect of Human Mesenchymal Stem Cells Is Mediated in Part from Secretion of the Antimicrobial Peptide LL-37. STEM CELLS 28, 2229-2238.
Luo, C., Zhang, F., Zhang, L., Geng, Y., Li, Q., Hong, Q., Fu, B., Zhu, F., Cui, S., Feng, Z., et al. (2014). Mesenchymal Stem Cells Ameliorate Sepsis-associated Acute Kidney Injury in Mice: Shock 41, 123-129.
Mei, S.H., McCarter, S.D., Deng, Y., Parker, C.H., Liles, W.C., and Stewart, D.J. (2007). Prevention of LPS-induced acute lung injury in mice by mesenchymal stem cells overexpressing angiopoietin 1. PLoS Med. 4, e269.
Mezey, E., Nemeth K. (2015). Mesenchymal stem cells and infectious diseases: Smarter than drugs. Immunol. Lett. 168(2), 208-214.
Moll, G., Geißler, S., Catar, R., Ignatowicz, L., Hoogduijn, M. J., Strunk, D., Bieback, K., and Ringdén, O. (2016). Cryopreserved or Fresh Mesenchymal Stromal Cells: Only a Matter of Taste or Key to Unleash the Full Clinical Potential of MSC Therapy? In F. Karimi-Busheri & M. Weinfeld (Eds.), Biobanking and cryopreservation of stem cells. (Vol. 951, Advances in Experimental Medicine and Biology., pp. 77-98). Cham, Switzerland: Springer.
Németh, K., Leelahavanichkul, A., Yuen, P.S.T., Mayer, B., Parmelee, A., Doi, K., Robey, P.G., Leelahavanichkul, K., Koller, B.H., Brown, J.M., et al. (2009). Bone marrow stromal cells attenuate sepsis via prostaglandin E2-dependent reprogramming of host macrophages to increase their interleukin-10 production. Nat. Med. 15, 42-49.
Pedrazza, L., Lunardelli, A., Luft, C., Cruz, C.U., de Mesquita, F.C., Bitencourt, S., Nunes, F.B., and de Oliveira, J.R. (2014). Mesenchymal stem cells decrease splenocytes apoptosis in a sepsis experimental model. Inflamm. Res. 63, 719-728.
Rojas, M., Cárdenes, N., Kocyildirim, E., Tedrow, J.R., Cáceres, E., Deans, R., Ting, A., and Bermúdez, C. (2014). Human adult bone marrow-derived stem cells decrease severity of lipopolysaccharide-induced acute respiratory distress syndrome in sheep. Stem Cell Res. Ther. 5, 42.
Wu, K.-H., Wu, H.-P., Chao, W.-R., Lo, W.-Y., Tseng, P.-C., Lee, C.-J., Peng, C.-T., Lee, M.-S., and Chao, Y.-H. (2015). Time-Series Expression of Toll-Like Receptor 4 Signaling in Septic Mice Treated with Mesenchymal Stem Cells: SHOCK 1.
Zhao, X., Liu, D., Gong, W., Zhao, G., Liu, L., Yang, L., and Hou, Y. (2014). The Toll-like Receptor 3 Ligand, Poly(I:C), Improves Immunosuppressive Function and Therapeutic Effect of Mesenchymal Stem Cells on Sepsis via Inhibiting MiR-143: Poly(I:C) Improves MSCs Immune Function. STEM CELLS 32, 521-533.
Mervyn Singer, Clifford S. Deutschman, Christopher Warren Seymour, Manu Shankar-Hari, Djillali Annane, Michael Bauer, Rinaldo Bellomo, Gordon R. Bernard, Jean-Daniel Chiche, Craig M. Coopersmith, Richard S. Hotchkiss, Mitchell M. Levy, John C. Marshall, Greg S. Martin, Steven M. Opal, Gordon D. Rubenfeld, Tom van der Poll, Jean-Louis Vincent, and Derek C. Angus. (2016). The Third International Consensus Definitions for Sepsis and Septic Shock (Sepsis-3). JAMA. 2016;315(8):801-810

## Revendications

1. Cellules souches mésenchymateuses (CSM) humaines décongelées issues de la gelée de Wharton pour leur utilisation dans le traitement du sepsis, notamment du choc septique.

2. Les CSM humaines décongelées issues de la gelée de Wharton pour leur utilisation selon la revendication **1, caractérisées en ce que** le niveau d'expression d'au moins un marqueur sélectionné parmi CD90, CD73, CD105, CD29, CD44, CD146, CD166, HLA-ABC est au moins 10% inférieur au niveau d'expression du même marqueur dans des CSM humaines fraîches issues de la gelée de Wharton.

3. Les CSM humaines décongelées issues de la gelée de Wharton pour leur utilisation selon la revendication **1** ou **2, caractérisées en ce que** le niveau d'expression du marqueur CD90 est au moins 10% inférieur au niveau d'expression du marqueur CD90 dans des CSM humaines fraîches issues de la gelée de Wharton.

4. Les CSM humaines décongelées issues de la gelée de Wharton pour leur utilisation selon l'une quelconque des revendications **1** à **3, caractérisées en ce que** lesdites CSM expriment au moins une protéine sélectionnée parmi le groupe comprenant ACTB, ANXA1, CAPZB, LASP1, PRDX2, PRDX3, PSA3, RS12 et SYWC.

5. Les CSM humaines décongelées issues de la gelée de Wharton pour leur utilisation selon l'une quelconque des revendications **1** à **4, caractérisées en ce que** lesdites CSM n'expriment substantiellement pas au moins une protéine sélectionnée parmi le groupe comprenant ACTS, AL1B1, ANX10, GBB1, GBB2, GPRIN1, DTNA, MIPO1, PSB3 et PSDE.

6. Les CSM humaines décongelées issues de la gelée de Wharton pour leur utilisation selon l'une quelconque des revendications **1** à **5, caractérisées en ce que** lesdites CSM, en condition *in vitro* et/ou en condition non-inflammatoire, sécrètent au moins un facteur de croissance sélectionné parmi BMP-7, IGFBP-1, insuline, FGF-7, NT-4 et VEGF-D.

7. Les CSM humaines décongelées issues de la gelée de Wharton pour leur utilisation selon l'une quelconque des revendications **1** à **6, caractérisées en ce que** lesdites CSM, en condition *in vivo* et/ou en condition inflammatoire, sécrètent au moins un facteur de croissance sélectionné parmi BMP-7 et TGFβ3.

8. Les CSM humaines décongelées issues de la gelée de Wharton pour leur utilisation selon l'une quelconque des revendications **1** à **7, caractérisées en ce que** lesdites CSM, en condition *in vivo* et/ou en condition inflammatoire, ne sécrètent substantiellement pas d'IGFBP-1.

9. Les CSM humaines décongelées issues de la gelée de Wharton pour leur utilisation selon l'une quelconque des revendications **1** à **8, caractérisées en ce que** lesdites CSM sécrètent au moins 1.2 fois plus de VEGF que des CSM humaines fraîches issues de la gelée de Wharton.

10. Les CSM humaines décongelées issues de la gelée de Wharton pour leur utilisation selon l'une quelconque des revendications **1** à **9, caractérisées en ce que** lesdites CSM induisent une augmentation de la concentration sérique de VEGF chez le patient d'au moins 5% par rapport à des CSM humaines fraîches issues de la gelée de Wharton.

11. Les CSM humaines décongelées issues de la gelée de Wharton pour leur utilisation selon l'une quelconque des revendications **1** à **10, caractérisées en ce que** lesdites cellules sont issues d'un tissu de cordon ombilical humain provenant d'une mère répondant à au moins l'un des critères suivants : ayant reçu une administration d'ocytocine lors de l'accouchement, ayant accouché par travail dirigé, ayant accouché à terme, n'ayant pas présenté de prééclampsie pendant la grossesse, dont l'enfant n'a pas présenté de troubles néonataux et ayant subi une imprégnation tabagique au cours de la grossesse.

12. Les CSM humaines décongelées issues de la gelée de Wharton pour leur utilisation selon l'une quelconque des revendications **1** à **11, caractérisées en ce que** lesdites CSM sont des cellules de grade clinique.

13. Les CSM humaines décongelées issues de la gelée de Wharton pour leur utilisation selon l'une quelconque des revendications **1** à **12, caractérisées en ce que** lesdites cellules sont directement issues de la décongélation sans remise en culture après la décongélation.

14. Une composition pharmaceutique comprenant des cellules souches mésenchymateuses humaines décongelées issues de la gelée de Wharton selon l'une quelconque des revendications **1** à **13** et un excipient pharmaceutiquement acceptable, pour son utilisation dans le traitement du sepsis.

15. Procédé de préparation de cellules souches mésenchymateuses de grade clinique issues de la gelée de Wharton, comprenant les étapes suivantes :
(i) cultiver un tissu de cordons ombilicaux humains contenant la gelée de Wharton dans un milieu de culture de grade clinique pour l'adhésion de cellules ;
(ii) incuber les cellules adhérentes dans un milieu contenant du lysat plaquettaire,
**caractérisé en ce que** ledit tissu provient d'une mère répondant à au moins l'un des critères suivants : ayant reçu une administration d'ocytocine lors de l'accouchement, ayant accouché par travail dirigé, ayant accouché à terme, n'ayant pas présenté de prééclampsie pendant la grossesse, dont l'enfant n'a pas présenté de troubles néonataux et ayant subi une imprégnation tabagique au cours de la grossesse.
